(19) Europäisches Patentamt
European Patent Office
Office européen des brevets

(11) EP 0 957 092 A1

(12) **EUROPÄISCHE PATENTANMELDUNG**

(43) Veröffentlichungstag:
**17.11.1999 Patentblatt 1999/46**

(21) Anmeldenummer: **99112223.5**

(22) Anmeldetag: **18.06.1993**

(51) Int. Cl.$^6$: **C07D 217/18**, C07D 471/04, C07D 495/04, A61K 31/47, A61K 31/435, C07D 217/14 // (C07D471/04, 221:00, 209:00), (C07D495/04, 333:00, 221:00)

(84) Benannte Vertragsstaaten:
**AT BE CH DE DK ES FR GB GR IE IT LI LU MC NL PT SE**

(30) Priorität: **22.06.1992 DE 4220353**
**22.06.1992 DE 4220319**
**22.06.1992 DE 4220355**
**22.06.1992 DE 4220368**
**22.06.1992 DE 4220345**
**22.06.1992 DE 4220312**
**22.06.1992 DE 4220373**
**22.06.1992 DE 4220369**

(62) Dokumentnummer(n) der früheren Anmeldung(en) nach Art. 76 EPÜ:
**93913009.2 / 0 647 220**

(71) Anmelder:
- **Boehringer Ingelheim Pharma KG**
**55216 Ingelheim am Rhein (DE)**
- **Boehringer Ingelheim International GmbH**
**55216 Ingelheim (DE)**

(72) Erfinder:
- **Arndts, Dietrich**
**55437 Appenheim (DE)**
- **Loesel, Walter**
**55435 Gau-Algesheim (DE)**
- **Roos, Otto**
**55270 Schwabenheim (DE)**

(74) Vertreter:
**Laudien, Dieter, Dr. et al**
**Boehringer Ingelheim GmbH**
**Abteilung Patente**
**55216 Ingelheim (DE)**

(54) **Anellierte Dihydropyridine und deren Verwendung für die Herstellung von pharmazeutischen Zubereitungen**

(57) Verbindung der allgemeinen Formel I oder deren Tautomeren der allgemeinen Formel II

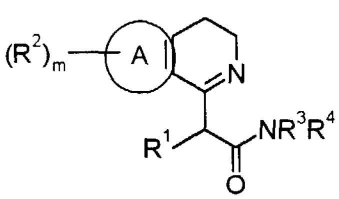

I

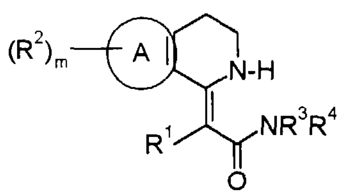

II

worin

A einen Benzo-, Indolo- oder Thienorest bedeutet;

$R_1$ $(C_4\text{-}C_6)$Cycloalkyl, $(C_4\text{-}C_6)$Cycloalkyl$(C_1\text{-}C_5)$alkyl oder

EP 0 957 092 A1

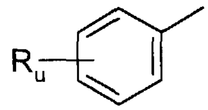

bedeutet,

$R^2$, m, $R^3$, $R^4$, R und u wie in der Beschreibung definiert sind und diese Verbindung enthaltende pharmazeutische Zubereitungen und deren neue pharmazeutische Verwendungen.

## Beschreibung

Die Erfindung betrifft anellierte Dihydropyridinessigsäurederivate, Verfahren zu ihrer Herstellung und diese Verbindungen enthaltende Arzneimittel.

Aus der EP-A 37 934 sind Dihydroisochinoline bekannt geworden. Die dort genannten Verbindungen sind cardioton wirksam und weisen eine kontraktilitätssteigernde und blutdruckbeeinflussende Wirkkomponente auf. Sie sind zur Verbesserung der Gewebsdurchblutung und der Gewebssauerstoffversorgung vorgeschlagen worden. Diese Verwendungsmöglichkeiten beruhen auf der vaskulären Wirksamkeit der Verbindungen. In der EP-A 251 194 ist beschrieben worden, daß carbocyclisch und heterocyclisch annelierte Dihydropyridine cardioprotektive Wirkung besitzen und einen völlig neuen Typ Ca-antagonistischer Verbindungen verkörpern.

Gegenstand der vorliegenden Erfindung sind neue carbocyclisch und heterocyclisch annelierte Dihydropyridine, und die pharmazeutische Verwendung dieser Verbindungen.

Ein Aspekt der Erfindung besteht in der Verwendung einer Verbindung der allgemeinen Formel I

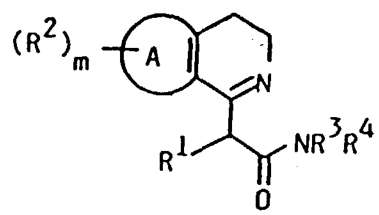

I

worin

A einen Benzo, Indolo oder Thienorest bedeutet; worin, wenn A Benzo ist, m 2 oder 3 ist, (vorzugsweise 2, wobei die beiden $R^2$ in den Positionen 6 und 7 sind) und die Substituenten $R^2$ unabhängig voneinander Hydroxy, $(C_1$-$C_4)$Alkoxy, Benzyloxy, Halogen (F, Cl, Br, J), $(C_1$-$C_4)$Alkyl, Methansulfonyloxy oder Methansulfonamido, oder zwei benachbarte Substituenten $R^2$ zusammen -0-$CH_2$-0- oder -0-$CH_2$-$CH_2$-O- sein können; und wenn A Indolo oder Thieno ist, m Null ist;

$R_1$ $(C_4$-$C_6)$Cycloalkyl, $(C_4$-$C_6)$Cycloalkyl$(C_1$-$C_5)$alkyl oder

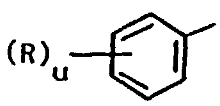

bedeutet,

$R^3$ und $R^4$ unabhängig voneinander

(a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeuten, wobei das Alkyl substituiert sein kann durch
Hydroxy,
$(C_1$-$C_4)$ Alkoxy,
Di $(C_1$-$C_4)$Alkylamino,
Furyl,
Pyridyl,
Pyrrolidinyl, N-Methylpyrrolidinyl,
Morpholino,
Indolyl,
Nitrilo,

Thienyl,
Adamantyl,
Cyclohexyl,
Phenoxy,
Naphthyloxy oder Phenyl, wobei dieses Phenyl oder das in der Phenoxygruppe enthaltene Phenyl ein-, zwei- oder dreifach durch Hydroxy, $(C_1$-$C_4)$Alkoxy, Benzyloxy, Halogen (F, Cl, Br, J), $CF_3$, $N_3$, $(C_1$-$C_4)$Alkyl, Aamantyl, $-SO_2NH_2$, $-NHCOCH_3$, $-NHSO_2CH_3$ oder $CH_3SO_2O$-oder durch die Brücke $-O-CH_2-O$-substituiert sein kann;
oder $R^3$ Wasserstoff bedeutet und $R^4$ Cyclohexyl, Phenyl, Fluorophenyl, Pyridyl oder N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl, die Gruppe

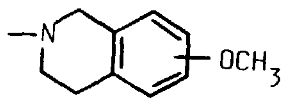

oder
Piperazinyl bedeuten, wobei der Piperazinylring gegebenenfalls durch Methyl, unsubstituiertes Phenyl, Mono- oder Di$(C_1$-$C_4)$alkoxyphenyl, Pyrimidinyl, Phenyl$(C_1$-$C_4)$ alkyl oder

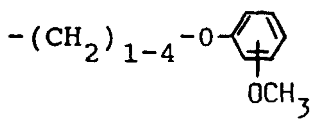

N-substituiert sein kann;

R für $C_1$-$C_4$-Alkyl, Hydroxy, $-N_3$, Halogen (F, Cl, Br, I), $CF_3$, $C_1$-$C_4$-Alkoxy oder -COH steht und

u für 0, 1, 2 oder 3;
oder deren Salze mit physiologisch verträglichen Säuren oder Komplexbildnern, zur Herstellung von Mitteln zur Behandlung chronisch inflammatorischer Prozesse, der Colitis Ulcerosa und des Morbus Crohn, und von Mitteln mit antiproliferativer Wirkung.

Ein anderer Aspekt der Erfindung besteht in folgenden neuen Verbindungen:

a) Neue Verbindung der allgemeinen Formel

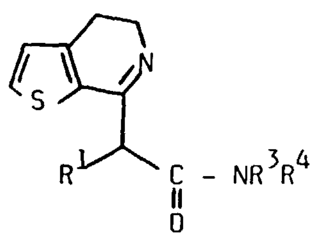

worin $R^1$, $R^3$ und $R^4$ wie oben definiert sind oder deren Salze mit physiologisch verträglichen Säuren oder Komplexbildern.

b) Neue Verbindung der allgemeinen Formel I

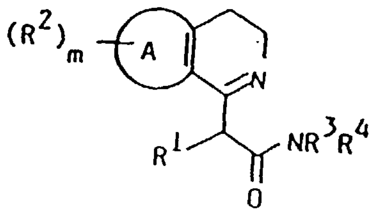

I

worin

A einen Benzo, Indolo oder Thienorest bedeutet; worin, wenn A Benzo ist, m 2 oder 3 ist, (vorzugsweise 2, wobei die beiden $R^2$ in den Positionen 6 und 7 sind) und die Substituenten $R^2$ unabhängig voneinander Hydroxy, $(C_1$-$C_4)$Alkoxy, Benzyloxy, Halogen (F, Cl, Br, J), $(C_1$-$C_4)$Alkyl, Methansulfonyloxy oder Methansulfonamido, oder zwei benachbarte Substituenten $R^2$ zusammen -0-$CH_2$-0- oder -0-$CH_2$-$CH_2$-O- sein können; und wenn A Indolo oder Thieno ist, m Null ist;

$R_1$ $(C_4$-$C_6)$Cycloalkyl, $(C_4$-$C_6)$Cycloalkyl$(C_1$-$C_5)$alkyl oder

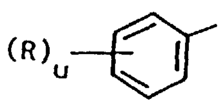

bedeutet,

$R^3$ und $R^4$ unabhängig voneinander

(a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeuten, wobei das Alkyl substituiert sein kann durch
Hydroxy,
$(C_1$-$C_4)$Alkoxy,
Di$(C_1$-$C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,
Cyclohexyl,
Phenoxy,
Naphthyloxy oder Phenyl, wobei dieses Phenyl oder das in der Phenoxygruppe enthaltene Phenyl ein-, zwei- oder dreifach durch Hydroxy, $(C_1$-$C_4)$Alkoxy, Benzyloxy, Halogen (F, Cl, Br, J), $CF_3$, $N_3$, $(C_1$-$C_4)$Alkyl, Aamantyl, -$SO_2NH_2$, -$NHCOCH_3$, -$NHSO_2CH_3$ oder $CH_3SO_2O$- oder durch die Brücke -O-$CH_2$-O- substituiert sein kann;
oder $R^3$ Wasserstoff bedeutet und $R^4$ Cyclohexyl, Phenyl, Fluorophenyl, Pyridyl oder N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl, die Gruppe

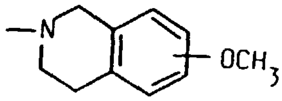

oder

Piperazinyl bedeuten, wobei der Piperazinylring gegebenenfalls durch Methyl, unsubstituiertes Phenyl, Mono- oder Di($C_1$-$C_4$)alkoxyphenyl, Pyrimidinyl, Phenyl($C_1$-$C_4$)alkyl oder

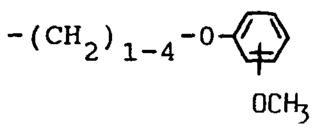

N-substituiert sein kann;

R für $C_1$-$C_4$-Alkyl, Hydroxy, -$N_3$, Halogen (F, Cl, Br, I), $CF_3$, $C_1$-$C_4$-Alkoxy oder -COH steht und

u für 1, 2 oder 3;

oder deren Salze mit physiologisch verträglichen Säuren oder Komplexbildnern.

Ein anderer Aspekt der Erfindung besteht in einzelnen neuen Verbindungen, die unter die allgemeine Definition älterer Patentanmeldungen fallen. Diese Verbindungen sind neben anderen Verbindungen in den Tabellen 13 bis 20, insbesondere den Tabellen 14, 16 und 20 enthalten. Folgende Verbindungen dieser Gruppe seien hervorgehoben:

Verbindung der Formel

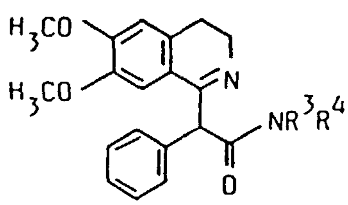

I

worin $NR^3R^4$ eine der folgenden Bedeutungen hat

Struktur

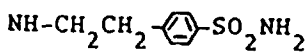 I

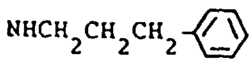 I

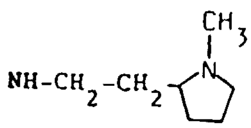 I

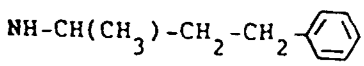 I+II

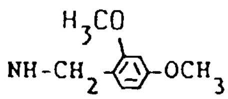 I

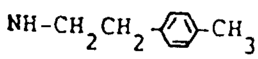 I

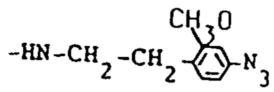

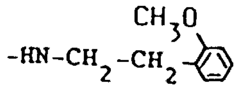

$-HN-CH_2-1-Adam.$

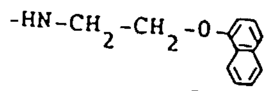

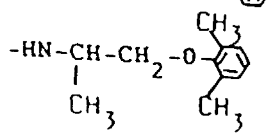

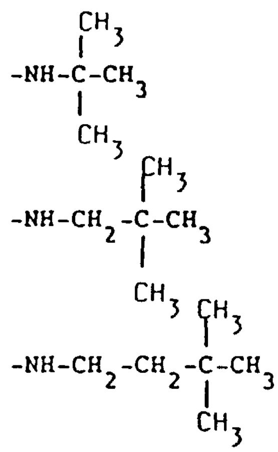

Struktur

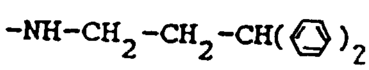 I

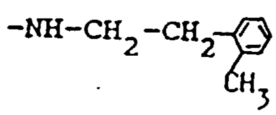 I

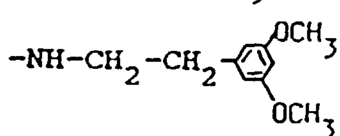 I

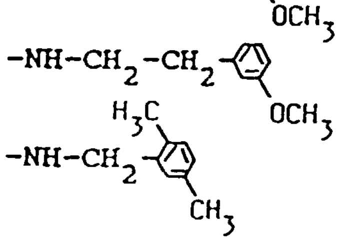 I

I

Struktur

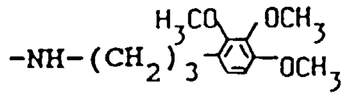
I

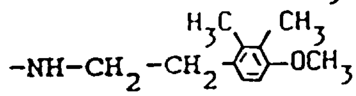
II

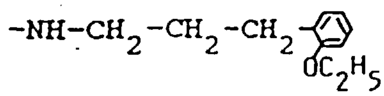
I

$-NH-CH_2-Adam(1)$
II

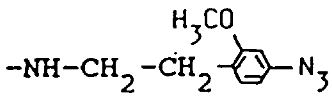
I

oder

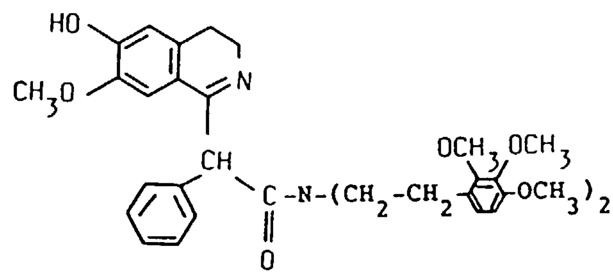

oder

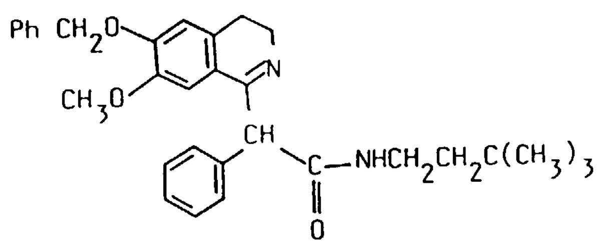

Verbindungen der Formel I bilden Tautomere der Formel II

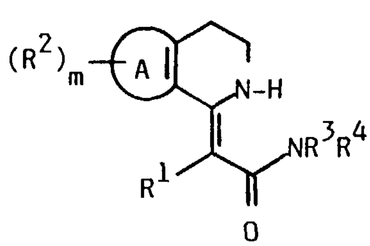
II

Die Tautomeren sind nach bekannten Methoden trennbar z.B. durch Säulenchromatographie oder selektive

Reduktion ($NaBH_4$ beziehungsweise katalytische Reduktion).

Die Verbindungen der Formel II können in cis und/oder trans-Form vorliegen:

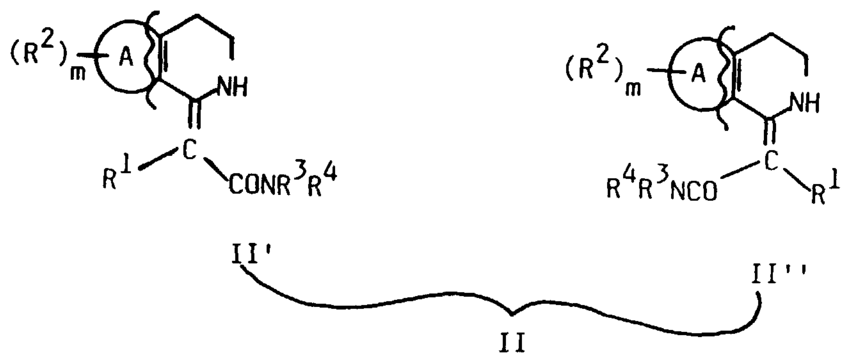

II' II'' II

Wenn die Struktur einer Verbindung nicht ausdrücklich angegeben ist, ist die Angabe der Formel I so zu verstehen, daß Struktur II ebenfalls umfaßt wird.

Die neuen Verbindungen haben wertvolle therapeutisch nutzbare Eigenschaften. Sie sind als cardioprotektive Mittel, als hirnprotektive Mittel (insbesondere bei der Behandlung von Patienten, die einen Schlaganfall erlitten haben oder gefährdet sind, einen Schlaganfall zu erleiden), als Mittel für die Behandlung chronisch inflammatorischer Prozesse (z.B. Bronchialasthma, Arthritis) verwendbar. Ferner können diese Verbindungen als Mittel mit antiproliferativer Wirkung und als Mittel zur Behandlung der Colitis Ulcerosa und des Morbus Crohn verwendet werden.

In den im Text verwendeten Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Mit Alkyl sind insbesondere $C_1$-$C_6$-Alkyl- und $C_1$-$C_4$-Alkylradikale gemeint die ihrerseits substituiert sein können oder als Alkylradikale Bestandteil einer funktionellen Gruppe - wie Alkoxy oder Alkylthio - sind. Die Alkylradikale umfassen die Methyl-, Ethyl-, n-Propyl-, iso-Propyl-, n-Butyl-, sek-Butyl-, iso-Butyl-, tert-Butylradikale sowie die verschiedenen isomeren Pentyl- und Hexylradikale, wie etwa das Isopentyl, das Neopentyl, das n-Pentyl und das n-Hexylradikal.

Die vorstehende Definition gilt somit auch wenn das Alkylradikal seinerseits substituiert und/oder selbst Bestandteil einer Alkoxyalkyl-, Alkoxycarbonyl-, Alkoxy-, Alkylthio-, Alkylsulfonyl-, Monoalkylamino-, Alkylmethyl-, Alkylthiomethyl-, Dialkylaminogruppe ist oder das Alkylradikal als Substituent an ein aromatisches heterocyclisches oder carboxyclisches System gebunden ist.

Halogene sind Fluor, Chlor, Brom und Jod, vorzugsweise Fluor, Chlor und Brom und in zweiter Linie Jod.

$C_3$-$C_6$-Cycloalkyl sind Cyclopropan, Cyclobutan, Cyclopentan und Cyclohexan.
$C_5$-$C_6$-Cycloalkene sind z.B. Cyclopenten, Cyclohexen und Cyclohexadien.

Das $C_2$- und $C_3$-Acylradikal steht für das Acetyl und das Propionylradikal.

$C_3$-$C_6$-Alkine sind die isomeren Hexine, Pentine, Butine und Propine bevorzugt ist Propargyl

$C_3$-$C_6$-Alkene sind die isomeren Hexene, Pentene, Butene und Propene bevorzugt ist Allyl

Als ungesättigte Heterocyclen sind unter anderem zu nennen:
Furan, Pyran, Pyrrol, Pyrazol, Imidazol, Pyridin, Pyrazin, Pyrimidin, Pyridazin, Thiophen, Thiazol, Oxazol, 1,2,4-Triazol, 1,2,3-Triazol, 1,2,4-Triazin, 1,3,5-Triazin, Indol.

Als 5- oder 6-gliedrige ganz oder teilweise gesättigte monocyclische Heterocyclen sind unter anderem zu nennen:

Imidazolidin, Pyrazolidin, Pyrrolidin, Piperidin, Piperazin, Morpholin, Thiomorpholin, Tetrahydrofuran, Tetrahydrothiophen, 1,4-Dioxin, Imidazolin, Pyrazolin, Pyrrolin, etc.

A. Von den Verbindungen der Formel I, worin $R^1$ ($C_4$-$C_6$)Cycloalkyl oder ($C_4$-$C_6$)Cycloalkyl($C_1$-$C_5$)alkyl ist, können folgende als besonders interessant hervorgehoben werden:
Verbindungen worin

$R^3$ und $R^4$ unabhängig voneinander (a) Wasserstoff, (b) Alkyl mit 1 bis 8 Kohlenstoffatomen, Alkenyl oder Alkinyl mit 2 oder 3 Kohlenstoffatomen (wobei das Alkyl substituiert sein kann durch Hydroxy, ($C_1$-$C_4$)Alkoxy, Di($C_1$-$C_4$)Alkylamino, Furyl, Pyrrolidinyl, Morpholinyl, Pyridinyl oder die Gruppe

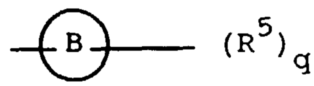

worin B, $R^5$ und q wie nachstehend definiert sind) (d) Dimethylamino, (f) Phenyl, (g) Morpholinyl, (h) Pyridyl bedeuten, wobei $R^3$ und $R^4$ nicht gleichzeitig Wasserstoff, Dimethylamino oder Di($C_1$-$C_4$)alkylaminomethyl sein können;
oder $R^3$ und $R^4$ gemeinsam mit dem Stickstoffatom, an das sie gebunden sind, einen Pyrrolidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl- ($C_1$-$C_4$)alkyl N-substituiert sein kann;
insbesondere worin $R^3$ und/oder $R^4$ unsubstituiertes Phenyl, Fluorphenyl, Morpholino oder 2- oder 3-Pyridyl bedeutet;
worin $R^3$ und/oder $R^4$ ($C_1$-$C_4$)Alkyl, vorzugsweise Methyl oder Ethyl, bedeutet; oder

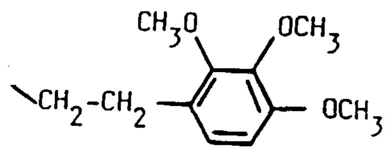

worin $R^3$ und/oder $R^4$ ($C_2$ oder $C_3$)Alkyl bedeutet, das durch Hydroxy, Methoxy, Dimethylamin, Furyl, Morpholino, Pyrrolidinyl oder Pyridinyl substituiert ist;
worin $R^3$ Wasserstoff ist.

Ferner sind von diesen Verbindungen (I) hervorzuheben, worin $R^3$ Wasserstoff ist und $R^4$ ein substituiertes Alkyl der Formel VII ist,

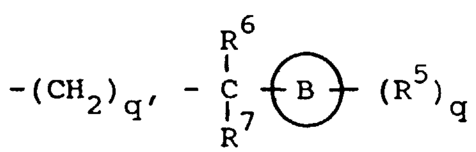

VII

worin q' O, 1 oder 2 ist;
$R^6$ und $R^7$ unabhängig voneinander Wasserstoff oder ($C_1$-$C_5$)Alkyl oder gemeinsam mit dem Kohlenstoffatom, an das sie gebunden sind, einen 5- oder 6-gliedrigen Carbocyclus bedeuten;
B Phenyl oder Thienyl bedeutet;
$R^5$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R^5$ zusammen -O-$CH_2$-O- oder -O-$CH_2$-$CH_2$-O- sind und
q' O, 1, 2 oder 3 bedeutet, wenn B Phenyl ist, und q', O, 1 oder 2 bedeutet, wenn B Thienyl ist;
insbesondere Verbindungen
worin $R^5$ ($C_1$-$C_4$)Alkyl, Hydroxy, ($C_1$-$C_4$)Alkoxy, Methansulfonyloxy oder Methansulfonamido bedeutet, oder zwei benachbarte Substituenten $R^5$ zusammen -O-$CH_2$-O- oder -O-$CH_2$-$CH_2$-O- sind;
worin $R^5$ Hydroxy, ($C_1$-$C_4$)Alkoxy, Methansulfonyloxy oder Methansulfonamido bedeutet, oder zwei benachbarte Substituenten $R^5$ zusammen -O-$CH_2$-O- oder -O-$CH_2$-$CH_2$-O- sind; insbesondere
worin $R^5$ Hydroxy, Methoxy, Methansulfonyloxy oder Methansulfonamido bedeuten, oder zwei benachbarte Substituenten $R^5$ zusammen -O-$CH_2$-O- sind;
besonders Verbindungen
worin $R^5$ Methoxy ist;
worin q null ist;

worin B Phenyl und q zwei ist, vorzugsweise worin die beiden Sustituenten $R^5$ in den Positionen 2 und 3 sind.

Hervorzuheben sind ferner Verbindungen, worin $R^3$ und $R^4$ gemeinsam mit dem Stickstoffatom, an das sie gebunden sind, Morpholino, Pyrrolidinyl oder Piperazinyl (das durch Methoxyphenyl, Phenethyl oder 2-Pyrimidinyl N-substituiert ist) bedeuten;

Von den genannten Verbindungsgruppen sind solche bevorzugt, worin

- $R^2$, Hydroxy, $(C_1-C_4)$Alkoxy, Methansulfonyloxy oder Methansulfonamido bedeutet, oder zwei benachbarte Substituenten $R^2$ zusammen $-O-CH_2-O-$ oder $-O-CH_2-CH_2-O-$ sind; insbesondere
- worin $R^2$ Methoxy ist.

B. Von den Verbindungen der Formel I, worin $R^1$

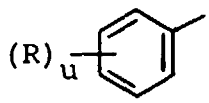

ist, können Verbindungen als besonders interessant hervorgehoben werden, worin

$R^3$ für Wasserstoff; und $R^4$ für Wasserstoff $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c), die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können), Phenoxy (worin die Phenylgruppe wie unter (b) angegeben substituiert sein kann),

b) Phenyl; gewünschtenfalls ein- oder mehrfach gleich oder verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe $-O-CH_2-O$ oder $-0-(CH_2)_2-0-$,

c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol (wobei die zuvor genannen Heterocyclen ein- oder mehrfach durch $C_1$-$C_4$-Alkyl substituiert sein können,) $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

oder $R^3$ Wasserstoff und $R^4$ Phenyl ist, das wie oben unter (b) angegeben substituiert sein kann;

$R^3$ und $R^4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;
oder

$R^3$ und $R^4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, Phenyl oder Benzyl substituiert sein kann (wobei diese Phenylgruppe oder die Phenylgruppe der Benzylgruppe wie vorstehend unter b) substituiert ist) steht.

Unter die beschriebene allgemeine Formel I fallen insbesondere 3,4-Dihydroisochinolinderivate
worin $NR^3R^4$

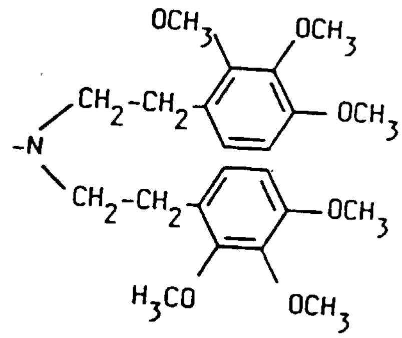

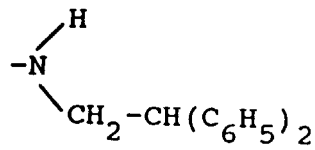

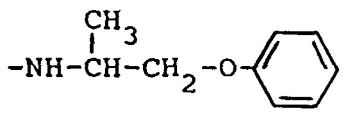

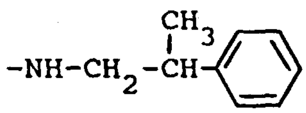

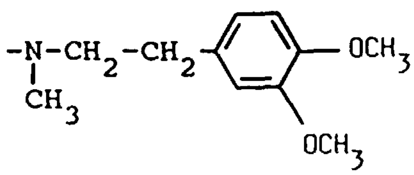

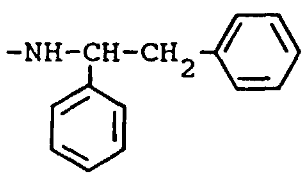

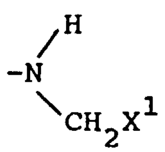

oder

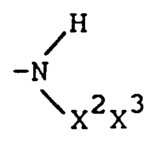

ist,
worin

$X^1$ durch Trifluormethyl oder Ethoxy mono- oder di-substituiertes Phenyl, durch Methoxy und Fluor substituiertes Phenyl oder 2-Methoxyphenyl ist,

$X^2$ -$CH_2$-$CH_2$- oder
-$CH_2$-$CH(CH_3)$- ist,
und

$X^3$ 2,3,4-Trimethoxyphenyl, 2,3-Dimethoxyphenyl, 2,6-Dimethoxyphenyl, 3,6-Dimethoxyphenyl, Thienyl, durch Trifluormethyl oder Ethoxy mono- oder di-substituiertes Phenyl, oder durch Methoxy und Fluor substituiertes Phenyl ist,

oder deren pharmazeutisch annehmbare Salze.

Bevorzugt sind Verbindungen (I), worin $NR^3R^4$

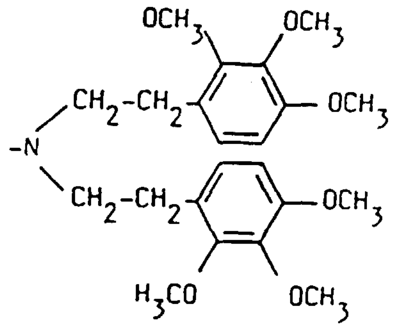

ist.

Bevorzugt sind carbocyclisch und heterocyclisch annelierte Dihydropyridine der Formel

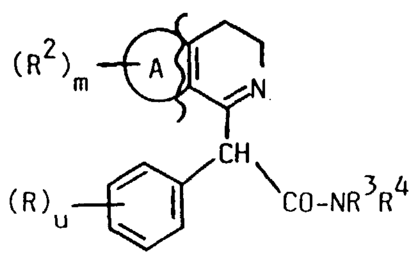

sowie deren tautomere Formen, worin

$R^3$ für Wasserstoff und

$R^4$ für Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$ Alkyl, welches einfach mit einem der in nachstehend genannten Substituenten der Gruppen d) bis f) substituiert ist;

d) Cyano, Hydroxy, Methoxy, Dimethylamino

e) Phenyl, 3,4-Methylendioxyphenyl, durch eine, zwei odere 3 Methoxygruppen substituiertes Phenyl, 3-Hydroxy-4-methoxyphenyl,

f) Morpholino, Pyridin-2-yl, Indol-3-yl, Furan-2-yl, Thiophen-2-yl, Pyridin-3-yl, Pyridin-4-yl

steht;

$R^3$ und $R^4$ unabhängig voneinander für Methyl; Ethyl; 3-Cyanopropyl; Benzyl; oder 3,4,5-Triniethoxyphenethyl stehen oder

$R^3$ und $R^4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;

und A für die anellierten Ringsysteme

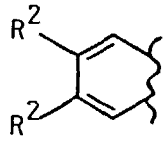

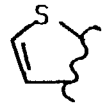

Der Phenylrest der Verbindung der allgemeinen Formel I kann 1, 2 oder 3 Substituenten R enthalten. Diese Substituenten R können gleich oder verschieden voneinander sein. Vorzugsweise ist R $C_1$-$C_4$-Alkyl (vorzugsweise Methyl), Halogen (vorzugsweise Fluor, Chlor oder Brom) oder $CF_3$.

C. Von den Verbindungen der Formel I, worin $R^1$ unsubstituiertes Phenyl ist, können Verbindungen als besonders interessant hervorgehoben werden, worin

$R^3$ Wasserstoff ist und

$R^4$ Wasserstoff, $C_3$-$C_6$-Alkenyl; $C_3$-$C_6$-Alkinyl; $C_3$-$C_6$-Cycloalkyl; $C_3$-$C_6$-Alkyl, das mit einer Benzogruppe anelliert ist; $C_3$-$C_6$-Cycloalkenyl; geradkettiges oder verzweigtes $C_1$-$C_{10}$-Alkyl, welches gewünschtenfalls ein- oder mehrfach mit den nachstehend genannten Substituenten der Gruppen a) bis c) die gleich oder verschieden sein können, substituiert ist:

a) Cyano; Hydroxy; $C_1$-$C_4$-Alkoxy; $C_1$-$C_4$-Alkylthio; $C_1$-$C_4$-Alkyloxycarbonyl; Amino; Mono-$C_1$-$C_4$-Alkylamino; Di-$C_1$-$C_4$-Alkylamino (wobei die Alkylradikale gleich oder verschieden sein können), Phenoxy (worin die Phenylgruppe wie unter (b) angegeben substituiert sein kann); Naphthoxy;

b) Phenyl; gewünschtenfalls ein-, zwei- oder dreifach gleich oder verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Azido, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe $-O-CH_2-O-$ oder $-0-(CH_2)_2-0-$;

c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol (wobei die zuvor genannen Heterocyclen ein- oder mehrfach durch $C_1$-$C_4$-Alkyl substituiert sein können)
$C_3$-$C_{10}$-Cycloalkyl (eventuell gebrücktes Cycloalkyl); $C_5$- oder $C_6$-Cycloalkenyl; $C_2$-$C_3$-Acyl; $C_1$-$C_4$-Alkylsulfonyl; oder $R^4$ Phenyl ist, das wie oben unter (b) angegeben substituiert sein kann;

$R^3$ und $R^4$ unabhängig voneinander je wie $R^4$ oben definiert sind, vorzugsweise für $C_1$-$C_6$-Alkyl, welches gewünschtenfalls phenylsubstituiert sein kann, wobei der Phenylsubstituent seinerseits wie vorstehend unter b) substituiert sein kann;
oder

$R^3$ und $R^4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_{q''}R^8$ (mit q'' = 0,1,2,3 oder 4) substituiert sein kann;
und

$R^8$ für ein Phenylradikal oder Phenoxyradikal, worin gewünschtenfalls die Phenylgruppe wie vorstehend unter b) substituiert ist, oder Naphthoxy steht;

A steht für die anellierten Ringsysteme

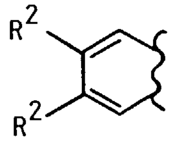

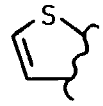

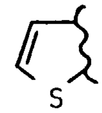

worin $R^2$ wie oben definiert ist,
insbesondere worin

$R^3$ Wasserstoff ist und

$R^4$ Wasserstoff; $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c), die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können), Phenoxy (worin die Phenylgruppe wie unter (b) angegeben substituiert sein kann),

b) Phenyl; gewünschtenfalls ein- oder mehrfach gleich oder verschieden substituiert durch die Gruppen Halogen, Trifluormethyl, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe -O-$CH_2$-O- oder -0-$(CH_2)_2$-0-

c) ein 5- oder 6-gliedriger gesättigter oder ganz oder teilweise ungesättigter monocyclischer Heterocyclus mit bis zu 3 Heteroatomen aus der Gruppe N, O, S; sowie als bicyclischer Heterocyclus Indol (wobei die zuvor genannen Heterocyclen ein- oder mehrfach durch $C_1$-$C_4$-Alkyl substituiert sein können,) $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

$R^3$ Wasserstoff ist und

$R^4$ Phenyl ist, das wie oben unter (b) angegeben substituiert sein kann;

$R^3$ und $R^4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;
oder

$R^3$ und $R^4$ zusammen mit dem Stickstoffatom, an welches sie gebunden sind, für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_{q''}$-$R^8$ (mit q" = 0 oder 1) substituiert sein kann
und

$R^8$ für ein Phenylradikal, welches gewünschtenfalls wie vorstehend unter b) substituiert ist, steht;

A steht für die anellierten Ringsysteme

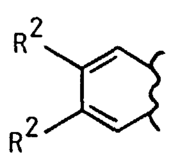

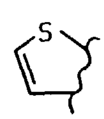

worin $R^2$ wie oben definiert ist.

Unter die beschriebene allgemeine Formel I fallen insbesondere auch 3,4-Dihydroisochinolinderivate der allgemeinen Formel

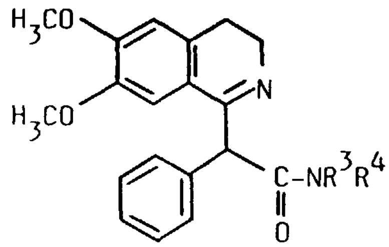

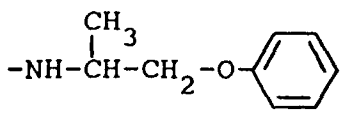

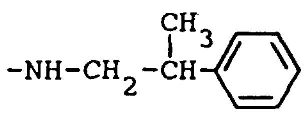

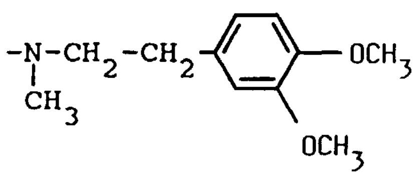

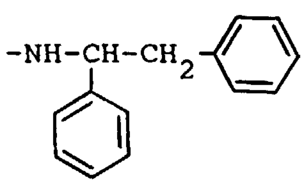

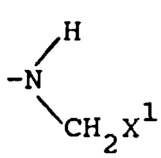

oder

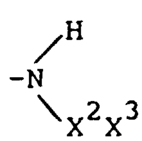

ist,
worin

$X^1$ durch Trifluormethyl oder Ethoxy mono- oder di-substituiertes Phenyl, durch Methoxy und Fluor substituiertes Phenyl oder 2-Methoxyphenyl ist,

$X^2$ $-CH_2-CH_2-$ oder $-CH_2-CH(CH_3)-$ ist, und

$X^3$ 2,3,4-Trimethoxyphenyl, 2,3-Dimethoxyphenyl, 2,6-Dimethoxyphenyl, 3,6-Dimethoxyphenyl, Thienyl, durch Trifluormethyl oder Ethoxy mono- oder di-substituiertes Phenyl, oder durch Methoxy und Fluor substituiertes Phenyl ist,

oder deren pharmazeutisch annehmbare Salze.

Bevorzugt sind insbesondere Verbindungen worin $NR^3R^4$

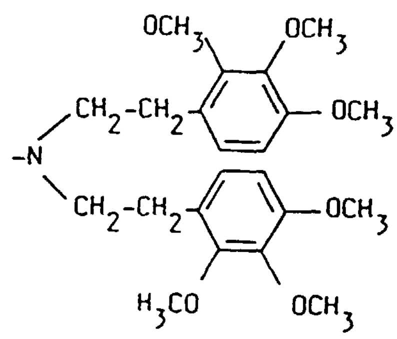

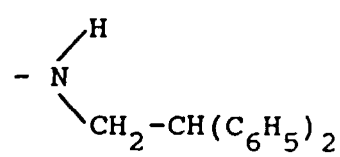

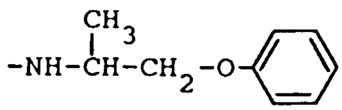

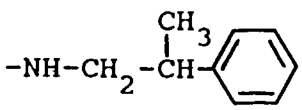

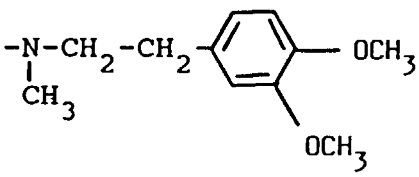

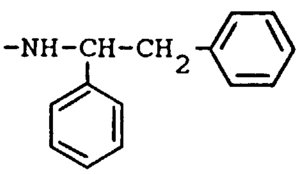

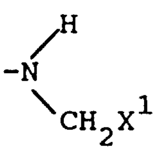

oder

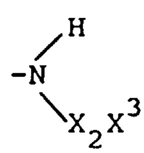

ist,
worin

X[1] 2-Methoxyphenyl ist, das zusätzlich durch Fluoro subsituiert sein kann,

X[2] $-CH_2-CH_2-$ ist
und

X[3] 2,3,4-Trimethoxyphenyl, 2,3-Dimethoxyphenyl, 2,6-Dimethoxyphenyl, 3,6-Dimethoxyphenyl, 2 oder 3-Thienyl, durch Trifluormethyl oder Ethoxy substituiertes Phenyl, oder durch Methoxy und Fluor substituiertes Phenyl ist.

Besonders hervorzuheben sind Verbindungen der Formel

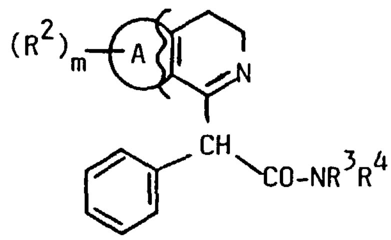

sowie deren tautomere Formen
worin

- $R^3$ Wasserstoff ist und
- $R^4$ Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;

  d) Cyano, Hydroxy, Methoxy, Dimethylamino

  e) Phenyl, 3,4-Methylendioxyphenyl, durch eine, zwei odere 3 Methoxygruppen substituiertes Phenyl, 3-Hydroxy-4-methoxyphenyl,

  f) Morpholino, Pyridin-2-yl, Indol-3-yl, Furan-2-yl, Thiophen-2-yl, Pyridin-3-yl, Pyridin-4-yl

- $R^3$ und $R^4$ unabhängig voneinander für Methyl; Ethyl; 3-Cyanopropyl; Benzyl; oder 3,4,5-Trimethoxyphenethyl stehen oder
- $R^3$ und $R^4$ zusammen mit dein Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen; und

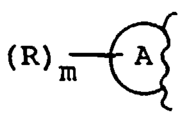

für die anellierten Ringsysteme

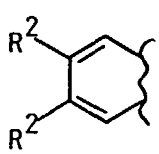

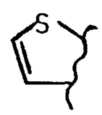

worin
$R^2$ wie oben definiert ist.

Die Verbindungen der Formel I können nach an sich bekannten Verfahren hergestellt werden, vorzugsweise nach der in der deutschen Patentanmeldung P 37 18 570.5, EP 358 957, EP 37 934 und EP 251 794 beschriebenen Methode.

In Gegenwart eines Kondensationsmittels kann ein Malonsäurediamid der allgemeinen Formel IV

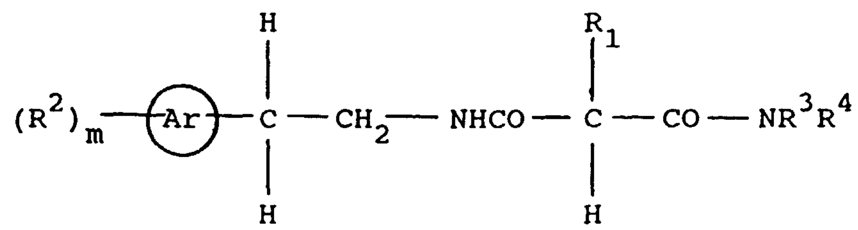

in der $R^1$, $R^2$, $R^3$, $R^4$ und m, wie oben definiert sind und Ar Phenyl oder 2- oder 3-Thienyl bedeutet, zu den entsprechenden Verbindungen cyclisiert werden.

Als Kondensationsmittel für dieses Verfahren eignen sich starke Lewis-Säuren, wie z.B. Phosphoroxychlorid, Phosphorpentachlorid, Phosphortrichlorid, Phosphorpentoxid, Titantetrachlorid, Bortrifluorid, Zinntetrachlorid, aber auch anorganische Säuren, wie z.B. Polyphosphorsäure, Schwefelsäure, Fluorsulfonsäure und Fluorwasserstoffsäure, oder Gemische von Kondensationsmitteln wie z.B. ein Gemisch von Phorphoroxychlorid und Phosphorpentachlorid, oder ein Gemisch von Phosphorpentoxid und ($C_1$-$C_4$) Alkylsulfonsäure z.B. mit einem $P_20_5$-Anteil von ca. 10 Gewichtsprozenten.

Die Cyclisierung kann in Gegenwart oder Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen, beispielsweise Benzol, Alkylbenzole (z.B. Toluol, Xylol), Chlorbenzole, Chloroform, Acetonitril, Dekalin. Eine bevorzugte Variante des Verfahrens besteht darin, das Kondensationsmittel, beispielsweise Phosphoroxychlorid oder ein ($C_1$-$C_4$)Alkylsulfonsäure-/Phosphorpentoxid-Gemisch, ohne Zusatz von Lösungsmitteln zu verwenden.

Bevorzugt erfolgt die Cyclisierung mit Phorphoroxychlorid oder in schwierigen Fällen mit einem Gemisch von Phosphorpentoxid und ($C_1$-$C_4$)Alkylsulfonsäure (bevorzugt Methansulfonsäure).
Die Umsetzung kann innerhalb eines großen Temperaturbereichs, vorzugsweise unter Erwärmen oder Erhitzen auf 50°C bis etwa den Siedepunkt des Reaktionsgemisches, durchgeführt werden.

Die erforderliche Reaktionsdauer liegt je nach Ausgangsverbindung IV zwischen 2 und 15 Stunden.

Die Verbindungen der Formel I sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Die Verbindungen können oral, parenteral oder topisch verabreicht werden. Der gewünschte therapeutische Dosis ist von der Indikation und Darreichungsform abhängig und kann experimentell bestimmt werden. Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Die Verbindungen können sowohl enteral als auch parenteral verabreicht werden. Als Dosis für die orale Anwendung werden 0,1 bis 500 mg Wirkstoff pro Dosis, für die i.v.-Anwendung von 0,05 bis 150 mg pro Dosis vorgeschlagen. Der gewünschte therapeutische Dosis ist von der Indikation und Darreichungsform abhängig und kann experimentell bestimmt werden.

Die Arzneimittel sind für orale oder parenterale, gegebenenfalls auch topische Verabreichung geeignet. Als Arzneimittelformen dienen vorwiegend Tabletten, Dragees, Ampullen und Saftzubereitungen. Die Einzeldosis zu diesen Arzneimittelformen beträgt zwischen 1,0 und 200 mg, vorzugsweise 20 und 50 mg pro 75 kg Körpergewicht. Je nach der Schwere des Falles sind täglich im allgemeinen 1 bis 3 Einzeldosen zu verabreichen.

Die folgenden Beispiele sollen die Erfindung näher erläutern:

Beispiel 1

3, 4-Dihydro-1-benzyl-6,7-dimethoxy-α-[di-2-(2,3,4-trimethoxyphenyl)ethyl]aminocarbonyl-isochinolinhydrochlorid

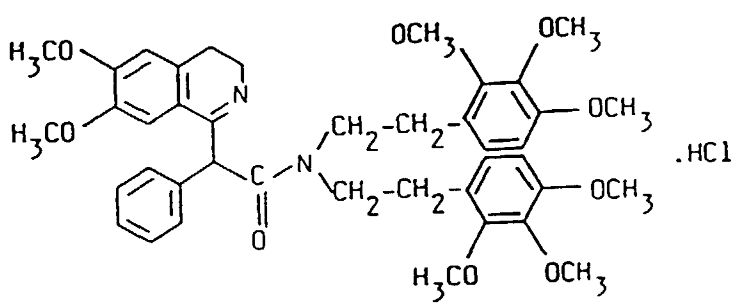

a) 2-(3,4-Dimethoxyphenyl)ethylaininocarbonyl-phenylessigsäure-N,N-di-[2-(2,3,4-trimethoxyphenyl)ethyl]amid

In eine Lösung von 18,0 g (52,4 mmol) Phenylmalonsäuremonoethylester-2-(3,4-dimethoxyphenyl)ethylamid in 150 ml wasserfreiem Dimethylformamid werden bei Raumtemperatur portionsweise 9,0 g (55,5 mmol) N,N'-Carbonyldiimidazol eingerührt. Nach 30 Min. werden 18,0 g (44,3 mmol) Di-[2-(2,3,4-Trimethoxyphenyl)ethyl]amin zugegeben und 30 Min gerührt. Danach wird das Lösungsmittel im Vakuum abdestilliert, der Rückstand in 1,5 l $CH_2Cl_2$ aufgenommen und nacheinander jeweils 2 mal mit 250 ml Wasser und 200 ml 1N HCl geschüttelt. Die organische Phase wird nach dem Trocknen über $Na_2SO_4$ eingeengt, und der Rückstand nach der Reinigung über eine Kieselgelsäule (Eluens: $CH_2Cl_2$/MeOH 100:2) aus Essigester/Ether kristallisiert. Ausbeute: 35,5 g

b) 35,0 g (47,5 mmol) Amid (aus Stufe a) und 15 ml (164 mmol) Phosphoroxychlorid werden in 150 ml wasserfreiem Acetonitril 30 Minuten zum Sieden erhitzt. Nach beendeter Umsetzung (Dünnschichtchromatographie-Kontrolle) werden das Lösungsmittel und nicht verbrauchtes Phosphoroxychlorid im Vakuum abdestilliert. Der Rückstand wird mit Eiswasser versetzt, mit Sodalösung alkalisch gestellt und mit ca. 1 l $CH_2Cl_2$ portionsweise extrahiert. Die organische Phase wird mit Wasser gewaschen, über $Na_2SO_4$ getrocknet und eingeengt. Der Rückstand wird zweimal über eine Kieselgelsäule (1. Eluens: $CH_2Cl_2$:MeOH 100:2 → 100:4 aufsteigend; 2. Eluens: $CH_2Cl_2$/Essigester 1:1) gereinigt.

Vom gereinigten Produkt (6,5 g) wird durch Lösen in ca. 50 ml Ethanol und Zugabe alkoholischer Salzsäure das Hydrochlorid gebildet. Nach dem Einengen und Trocknen im Hochvakuum bei 50°C verbleiben 11,5 g des gewünschten Produkts. (Fp. 56-64°C, amorph)

Beispiel 2

2-Phenylmalonsäure-N-(2-(3,4-dimethoxyphenyl)ethyl)-N',N'-di-(2-(2-Fluorphenyl)ethyl)-diamid

In eine Lösung von 17,2 g (0,05 mol) Phenylmalonsäure N-(2-(3,4-dimethoxyphenyl)ethyl)-monoamid in 200 ml wasserfreiem $CH_2Cl_2$ werden bei Raumtemperatur und unter Rühren 8,1 g (0,05 mol) N,N'-Carbonyldiimidazol portionsweise eingetragen. Nach ca. 30 min. wird in das Reaktionsgemisch eine Suspension bestehend aus 14,9 g (0,05 mol) Di-(2-(2-Fluorphenyl)ethyl)amin-hydrochlorid und 5,1 g = 6,4 ml (0,05 mol) Triethylamin in 100 ml wasserfreiem $CH_2Cl_2$ eingerührt. nach 15 stündigem Rühren werden 200 ml Wasser zugegeben, es wird mit verdünnter Salzsäure angesäuert, die org. Phase abgetrennt und die wasserphase 3x mit je 100 ml $CH_2Cl_2$ extrahiert. Die vereinigten org. Phasen. werden über $Na_2SO_4$ getrocknet und eingeengt.

R,S (3,4-Dihydro-6,7-dimethoxyisochinolin-1-yl)-2-phenyl N,N-di-(2-(2-fluorphenyl)ethyl-acetamid-oxalat

Ein Gemisch aus 29,8 g (0,049 mol) Amid, 150 ml wasserfreiem $CH_2Cl_2$ und 17,5 g = 9,4 ml (0,10 mol) $POCl_3$ wird 10 Stunden am Rückfluß erhitzt. Nach beendeter Umsetzung (DC-Kontrolle) wird der Ansatz in ein Gemisch aus 400 ml Eiswasser und 200 ml $CH_2Cl_2$ eingerührt. Danach wird mit einer gesättigten Sodalösung neutralisiert, die organische Phase wird abgetrennt, die wässrige 3x mit je 100 ml $CH_2Cl_2$ extrahiert. Die vereinigten Phasen werden über $Na_2SO_4$ getrocknet und i.v. eingeengt. Der Rückstand wird nach dem Reinigen an einer Kieselsäure (Eluens: $CH_2Cl_2$ = Methanol = 100:2) in wenig Ethanol gelöst und durch Zugabe einer stöchiometrischen Menge einer alkoholischen Oxalsäurelösung das Oxalat überführt. Das Oxalat wird auch durch Zugabe von Ether zur Kristallisation gebracht. Fp: 144-146°C.

Beispiel 3

(R,S)-(3,4-Dihydro-6,7-dimethoxyisochinolin-1-yl)-2-(4-methoxyphenyl)-N,N-di(2-(2,3,4-trimethoxyphenyl)ethyl)-acetamid-oxalat

Einsatz:

5 g (6,57 mMol) 4-Methoxyphenylmalonsäure-N-(2-(3,4-dimethoxyphenyl)ethyl)-N'-(2-(2,3,4-trimethoxyphenyl)-ethyl-diamid ("Diamid")
18 ml Acetonitril, 3,02 g (19,7 mMol) Phosphoroxychlorid 600 mg (6,66 mMol) Oxalsäure, wasserfrei, 200 ml Ether
Durchführung: Das Reaktionsgemisch aus "Diamid", Acetonitril und Phosphoroxychlorid wird unter $N_2$-Schutz 1 Stunde bei Rückflußtemperatur erhitzt. Nach dem Abkühlen mit Eiswasser wird mit in 100 ml Essigester verdünnt und nacheinander je 2 mal mit Eiswasser, 50 ml gesättigter $NaHCO_3$-Lösung, Wasser und gesättigter NaCl-Lösung gewaschen. Die organische Phase wird über $MgSO_4$ getrocknet und unter Rühren in eine Lösung von 706 mg (7,84 mMol) wasserfreier Oxalsäure in 200 ml abs. Ether gegossen.

Das Reaktionsprodukt schied sich als Oxalsäuresalz zunächst als Öl ab, welches nach einigem Stehen unter Rühren durchkristallisierte. Nach Stehen über Nacht im Eisschrank wird abgesaugt, mit Ether gewaschen und getrocknet. Fp: 107-110°C
Die Struktur wurde mit NMR-Spektroskopie bestätigt.

$R_f$-Werte: 0,53 (Essigester)
$R_f$-Werte: 0,6 (Acetonitril:$H_2O$ = 9:1)

Herstellung der Ausgangsverbindung:

Teil A

4-Methoxyphenylmalonsäurediethylester

Einsatz:

150 ml abs. Ethanol
7,5 g (0,33 Mol) Natrium
300 ml Diethylcarbonat 62,5 g (0,3 Mol) 4-Methoxyphenylessigsäureethylester

Durchführung:
Natrium wird in abs. Ethanol gelöst und im Vakuum zur Trockne eingedampft. Unter Eiswasserkühlung wird der Rückstand unter Rühren mit Diethylcarbonat und 3-Chlorphenylessigsäureethylester versetzt. Danach wird das Ethanol langsam (2-3 Stunden) über eine 40 cm-Kolonne (Raschigringe) im Hoch-Vakuum bei 40-70°C und 200 mM abdestilliert. Nach Abkühlen wird mit 30 ml Eisessig angesäuert und mit 150 ml Wasser versetzt. Das ausgeschiedene Öl wird nacheinander mit Wasser und gesättigter NaCl-Lösung gewaschen und über $MgSO_4$ getrocknet.
Der Rückstand wird an der Ölpumpe im Kugelrohr destilliert; Kp. 0,5 mM. : 150 - 155°C.

4-Methoxyphenylmalonsäuremonoethylester

Einsatz:

52,2 g (0,196 Mol) Diethylester
120 ml Ethanol
120 ml Wasser
12,3 g (0,22 Mol) KOH in 60 ml Wasser und 60 ml Ethanol

Durchführung:
Eine ethanolische Lösung von 4-Methoxyphenylmalonsäurediethylester wird unter Rühren und Eiskühlung mit der wässrig, alkoholischen KOH-Lösung versetzt und 75 Minuten gerührt. Danach wird mit einer gesättigten Zitronensäurelösung angesäuert und 3 mal mit Methylenchlorid extrahiert. Die organische Phase wird nacheinander mit Wasser und gesättigter NaCl-Lösung gewaschen, über $MgSO_4$ getrocknet und das Lösungsmittel im Vakuum abdestiliert. Der kristalline Rückstand wird aus Methylenchlorid/Petrolether (40-800) umkristallisiert.
Ausbeute: 34,4 g (73,6% der Theorie).
Die Struktur wurde durch NMR-Spektroskopie bestätigt.

N-(2-3,4-Dimethoxyphenyl)ethyl)-4-methoxy-phenylmalonsäure-monoethylester-amid

Einsatz:

34,4 g (0,144 Mol) 4-Methoxy-phenylmalonsäuremonoethylester
150 ml Tetrahydrofuran wasserfrei
23,3 g (0,144 Mol) N,N'-Carbonyldiimidazol
26,1 g (0,144 Mol) 2-(3,4-Dimethoxyphenyl)ethylamin in
50 ml Tetrahydrofuran wasserfrei

Durchführung:
In die Lösung des 4-Methoxy-phenylmalonsäurehalbesters in THF wird bei 50°C unter Rühren das Carbonyldiimidazol portionsweise eingetragen. Nach 30 Minuten Rühren bei Raumtemperatur wird unter Eiskühlung das Amin zugesetzt und 16 Stunden bei Raumtemperatur gerührt. Das Reaktionsgemisch wird eingeengt und der Rückstand in $CH_2Cl_2$ aufgenommen. Es wird je 2 mal mit Wasser, dann mit 10 %iger $KHSO_4$-Lösung, gesättigter $NaHCO_3$-

Lösung, Wasser und gesättigter Kochsalzlösung gewaschen. Nach dem Trocknen über $MgSO_4$ wird die organische Phase eingeengt und der ölige Rückstand (7,7 g) aus Essigester kristallisiert.

4-Methoxy-phenylmalonsäure-N-(2(3,4-dimethoxyphenyl)ethyl)-amid

Einsatz:

44,16 g (0,11 Mol) (4-Methoxy-phenylmalonsäurehalbesteramid
300 ml Methanol
120 ml (0,12 Mol) 1N Natronlauge

Durchführung:
In die methanolische Lösung des Halbesteramids wird bei 5-10°C während 30 Minuten die Natronlauge eingerührt. Nach 3-stündigem Rühren bei Raumtemperatur wird eingeengt, mit Wasser verdünnt, anschließend 2 mal mit $CH_3Cl$ extrahiert. Die wässrige Phase wird mit konzentrierter HCl auf pH=1 gestellt und mit $CH_3Cl$ 2 mal extrahiert. Nach dem Waschen mit gesättigter Kochsalzlösung wird die organische Phase über $MgSO_4$ getrocknet und eingeengt. Der kristalline Rückstand wird aus $CH_3Cl$ kristallisiert.

Teil B

2-(2,3,4-Trimethoxyphenyl)-1-nitro-ethern

Einsatz:

400 g (2,04 mol) 2,3,4-Trimethoxybenzaldehyd
1740 ml Eisessig
172,6 g (2,24 mol) Ammoniumacetat (wasserfrei)
656 ml Nitromethan

Durchführung:
Das Gemisch aus Benzaldehyd, Ammonacetat, Nitromethan und Eisessig wird unter Stickstoffschutz 30 Minuten bei Siedetemperatur geruhrt. Man kühlt auf -5°C ab, gießt unter Ruhren in 5 l Eiswasser. Der zähe Rückstand wird mit $CH_2Cl_2$ erschopfend extrahiert. Die organische Phase wird mit Wasser gewaschen, uber $MgSO_4$ getrocknet und eingeengt. Der braune, ölige Rückstand (510 g) kristallisiert beim Stehen. Er wird in 470 ml Essigester gelöst und durch Zugabe von 3,9 l Cyclohexan zur Kristallisation gebracht.

2-(2,3,4-Trimethoxyphenyl)ethylamin-hydrochlorid

Einsatz:

144,5 g (0,60 mol) "Nitrostyrol"
1,9 l Wasser
232 ml konz. Salzsäure (p.A.)
88 g PdC (10 %ig)

Durchführung:
Das obige Reaktionsgemisch wird bei 60°C, 35 bar, 2,25 Stunden hydriert. Danach wird im Vakuum eingeengt, der Rückstand in Ethanol aufgenommen und zur Trockne eingedampft. Danach wird in Ethanol gelöst und das Umsetzungsprodukt durch Zugabe von Ether zur Kristallisation gebracht.

2,3,4-Trimethoxybenzylalkohol

Einsatz:

500 g (2,55 mol) 2,3,4-Trimethoxybenzaldehyd
5,0 l Methanol
13,0 g $PtO_2$

Durchführung:
Die Reduktion wird bei 20°C und 5 bar durchgeführt und ist nach 30 min beendet. Nach dem Abdampfen des Lösungsmittels wird der Rückstand (501,5 g) im Hochvakuum destilliert (Kp 116°; 0,5 mbar).

2,3,4-Trimethoxybenzylchlorid

Einsatz:

50,0 g (0,25 mol) 2,3,4-Trimethoxybenzylalkohol
800 ml Methylenchlorid wasserfrei
59,4 g (0,5 mol) Thionylchlorid

Durchführung:
Die Losung des Alkohols in wasserfreiem $CH_2Cl_2$ wird unter Rühren und Eis-Kochsalz-Kühlung langsam mit $SOCl_2$ versetzt. Man rührt 15 weitere Minuten in der Kälte, dann 2 Stunden bei Raumtemperatur. Lösungsmittel und überschüssiges Thionylchlorid werden im Vakuum entfernt, der Rückstand wird in $CH_2Cl_2$ aufgenommen und nacheinander mit gesättigter $NaHCO_3$-Lösung, Wasser und gesättigter Kochsalzlösung geschüttelt. Nach dein Trocknen über $MgSO_4$ wird das Lösungsmittel im Vakuum abgezogen und der Rückstand im Kugelrohrofen an der Ölpumpe destilliert (Kp 118°C; 0,1 mbar).

2,3,4-Trimethoxybenzylcyanid

Einsatz:

75,8 g (0,35 mol) 2,3,4-Trimethoxybenzylchlorid
700 ml Aceton wasserfrei
3,45 g (0,023 mol) NaJ
25,7 g (0,53 mol) trockenes, pulverisiertes NaCN

Durchführung:
Das Reaktionsgemisch aus Benzylchlorid, NaJ und NaCN in wasserfreiem Aceton wird 20 Stunden bei Siedetemperatur gerührt. Nach dem Abkühlen wird abgesaugt und das Lösungsmittel im Vakuum entfernt. Der Rückstand wird in Essigester gelöst, zuerst mit Wasser, dann mit gesättigter Kochsalzlösung geschüttelt und über $MgSO_4$ getrocknet. Nach dem Entfernen des Lösungsmittels wird der Rückstand im Kugelrohrofen bei 0,015 mbar (Kp: 135°C) destilliert.

2,3,4-Trimethoxyphenylessigsäure

Einsatz:

138,5 g (0,67 mol) 2,3,4-Trimethoxybenzylcyanid,
53,5 g (1,34 mol) NaOH gelöst in 215 ml Wasser

Durchführung:

Das Gemisch aus Benzylcyanid und wässriger Natronlauge wird 7 Stunden bei Rückflußtemperatur gerührt, nach dem Abkühlen mit 6N $H_2SO_4$ angesäuert und 3 mal mit $CH_2Cl_2$ extrahiert. Die organische Phase wird nach dem Waschen mit Wasser und gesättigter NaCl-Losung über $MgSO_4$ getrocknet. Nach dein Entfernen des Lösungsmittels wird der Rückstand in 200 ml $CH_2Cl_2$ gelöst und durch Zugabe von 1500 ml Cyclohexan zur Kristallisation gebracht.

2,3,4-Trimethoxyphenyl-N-(2-(2,3,4-trimethoxyphenyl)ethyl)-acetamid

Einsatz:

72,4 g (0,32 mol) 2,3,4-Trimethoxyphenylessigsäure
400 ml wasserfreies Tetrahydrofuran
51,8 g (0,32 mol) N,N'-Carbonyldiimidazol (CDI)
79,3 g (0,32 mol) 2-(2,3,4-Trimethoxyphenyl)ethylalminhydrochlorid
500 ml wasserfreies Tetrahydrofuran
32,4 ml (0,32 mol) Triethylamin

Durchführung:

Die in wasserfreiem THF gelöste Phenylessigsäure wird unter Rühren bei 5°C durch portionsweise Zugabe von CDI in das Imidazolid überfuhrt. Nach 30 min wird bei Raumtemperatur eine Suspension von Aminhydrochlorid, Triethylamin in wasserfreiem THF eingerührt. Nach 16 Stunden wird im Vakuum eingeengt und der Rückstand zwischen $CH_2Cl_2$ und 2N HCl verteilt. Darauf wird nacheinander mit Wasser, gesättigter $NaHCO_3$-Lösung, Wasser und gesättigter NaCl-Lösung gewaschen. Nach dem Trocknen uber $MgSO_4$ wird die organische Phase eingeengt, der Rückstand wird in 200 ml Essigester gelöst und das Produkt durch Zusatz von 700 ml Cyclohexan zur Kristallisation gebracht.

Di-2-(2,3,4-Trimethoxyphenyl)ethylamin-hydrochlorid

Einsatz:

113,4 g (0,27 mol) "Phenylessigsäureamid"
470 ml wasserfreies Tetrahydrofuran
270 ml (0,54 mol) $BH_3 \cdot S(CH_3)_2$ in THF (2 mol/l)

Durchführung:

In die Lösung des Säureamids in wasserfreiem THF wird bei 65°C das Borankomplex-Gemisch unter $N_2$-Schutz zugetropft. Nach beendeter Zugabe wird weitere 15 min bei 65°C gerührt. Danach wird das Reaktionsgemisch auf 5°C abgekühlt. Es wird mit methanolischer Salzsäure vorsichtig angesäuert, im Vakuum (Abzug) eingeengt und der Rückstand aus Ethanol unter Etherzusatz kristallisiert.

Teil C

4-Methoxy-Phenylmalonsäure-N-(2-(3,4-dimethoxyphenyl)ethyl)-N'-(2-(2,3,4-trimethoxyphenyl)ethyl)-di-amid

Einsatz:

3,73 g (10 mMol) 4-Methoxy-Phenylmalonsäurehalbamid
25 ml wasserfreies THF
1,62 g (10 mMol) N,N'-carbonyldiimidazol
4,1 g (10 mMol) Di-2-(2,3,4-trimethoxyphenyl)ethylaminhydrochlorid
40 ml wasserfreies THF

1,01 g (10 mMol) Triethylamin = 1,39 ml

Durchführung:

In die Lösung des Monoamids in THF wird bei 5°C unter Rühren das Carbonyldiimidazol portionsweise eingetragen. Das Reaktionsgemisch wird 30 Minuten bei Raumtemperatur gerührt. Anschließend wird unter Eiskühlung mit einer Suspension des Amin-hydrochlorid in THF und Triethylamin umgesetzt. Nach 16-stündigem Rühren bei Raumtemperatur wird eingeengt und der Rückstand in Essigester gelöst. Die organische Phase wird nacheinander mit Wasser, 5 %iger $KHSO_4$-Lösung, gesättigter $HaHCO_3$-Lösung, Wasser und ungesättigter NaCl-Lösung gewaschen. Nach dem Trocknen über $MgSO_4$ wird eingeengt, der Rückstand (7,2 g) an 210 g Kieselgel (Eluens: Essigester/n-Hexan=2:1) gereinigt.

Die folgenden Tabellen fassen Beispiele von erfindungsgemäßen Verbindungen zusammen, wobei die Tabellen 10-20 neue Verbindungen zusammenfassen.

Tabelle 1

Strukturtyp:

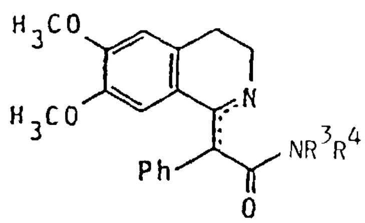

| Nr. | $NR^3R^4$ | Struktur | Salzform |
|---|---|---|---|
| 3. | $NHCH_3$ | I | Cl |
| 4. | $NHC_2H_5$ | I | - |
| 5. | $NH-(CH_2)_2-CH_3$ | I | Cl |
| 6. | $NH-(CH_2)_3-CH_3$ | I | Cl |
| 7. | $NH-(CH_2)_4-CH_3$ | I | Cl |
| 8. | $NH-CH(CH_3)_2$ | I | Cl |
| 9. | $NH-CH_2-CH(CH_3)_2$ | I | Cl |
| 10. | $NH-(CH_2)_2-CH(CH_3)_2$ | I | Cl |
| 11. | $NH-C(CH_3)_3$ | I | Cl |
| 12. | $NH-CH(CH_3)-C_2H_5$ | I | Cl |
| 13. | $NH-CH_2-CH=CH_2$ | I | Cl |
| 14. | $NH-CH_2-C\equiv CH$ | I | Cl |
| 15. | $NH-(CH_2)_2-OH$ | II | Cl |
| 16. | $NH-CH_2-CH(OH)-CH_3$ | I | Cl |
| 17. | $NH-(CH_2)_2-OCH_3$ | II | Cl |
| 18. | $NH-(CH_2)_3-OCH_3$ | II | Cl |
| 19. | $NH-(CH_2)_2-N(CH_3)_2$ | I | $Cl_2$ |
| 20. | $NH-(CH_2)_3-N(CH_3)_2$ | II | $Cl_2$ |
| 21. | $NH-(CH_2)_2-$ 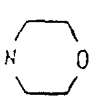 | II | - |

| Nr. | $NR^3R^4$ | Struktur | Salzform |
|---|---|---|---|
| 22. | 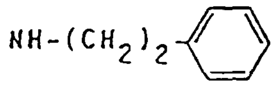 | I | Cl |
| 23. | 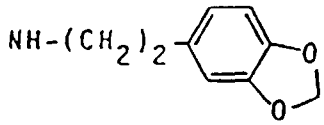 | I | - |
| 24. | 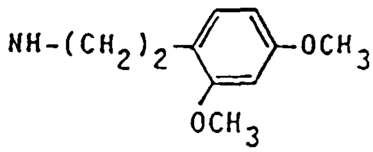 | I | - |
| 25. | 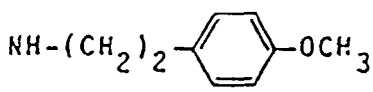 | I | - |
| 26. | 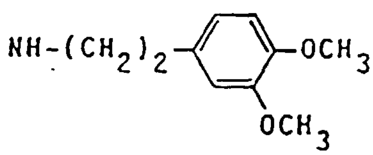 | II | - |
| 27. | 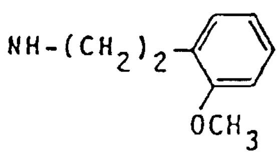 | II | Cl |
| 28. | 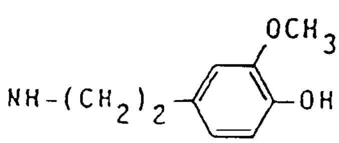 | I | - |
| 29. | 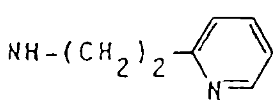 | I | - |

| Nr. | X | Struktur | Salzform |
|---|---|---|---|
| 30. | 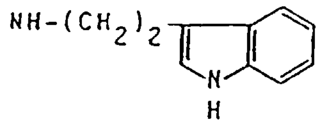 | II | - |
| 31. | 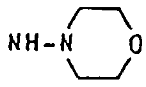 | I | - |
| 32. | 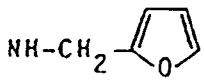 | II | - |
| 33. | 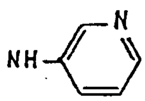 | I | Cl |
| 34. | 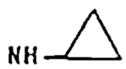 | I | Cl |
| 35. | 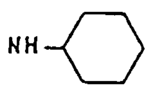 | II | - |
| 36. | $N(CH_3)_2$ | I | Cl |
| 37. | $N(C_2H_5)_2$ | I | Cl |
| 38. | 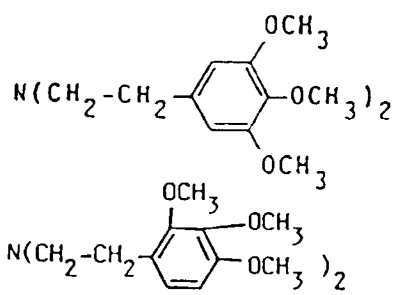 | I | Cl |
| 38a. |  | I | Cl |
| 39. | 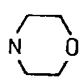 | I | Cl |

| Nr. | $NR^3R^4$ | Struktur | Salzform |
|---|---|---|---|
| 40 | 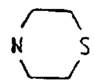 | I | - |
| 41. | 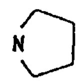 | I<br>II | - |
| 42. | 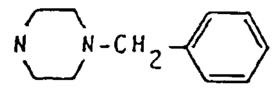 | II | $Cl_2$ |
| 43 | 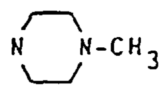 | I | - |
| 44 | 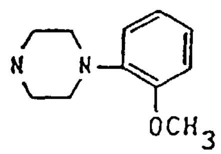 | I | Cl |

Tabelle 2

Strukturtyp:

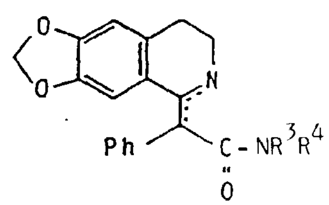

| Nr. | $NR^3R^4$ | Struktur | Salzform |
|---|---|---|---|
| 46. | $NH-(CH_2)_2-$ 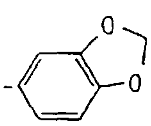 | II | Cl |
| 47. | $NH-(CH_2)_2$ 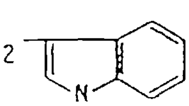 | II | - |

| Nr. | $NR^3R^4$ | Struktur | Salzform |
|---|---|---|---|
| 48. | NH-(CH$_2$)$_2$- 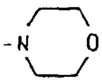 | II | - |
| 49. | 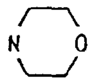 | II | - |
| 50. | 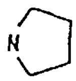 | I | - |

Tabelle 3

Strukturtyp:

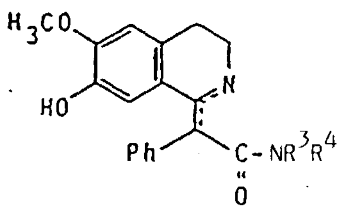

| Nr. | $NR^3R^4$ | Struktur | Salzform |
|---|---|---|---|
| 52. | $NH-(CH_2)_3-CH_3$ | I | - |
| 53. | $NH-(CH_2)_4-CH_3$ | I | - |
| 54. | $NH-CH(CH_3)_2$ | I | Cl |
| 55. | $NH-CH_2-CH(CH_3)_2$ | I | - |
| 56. | $NH-CH_2-CH=CH_2$ | I | - |
| 57. | NH-(CH$_2$)$_2$- 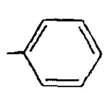 | I | Cl |
| 58. | NH-(CH$_2$)$_2$- 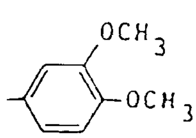 | I | Cl |

| Nr. | $NR^3R^4$ | Struktur | Salzform |
|---|---|---|---|
| 59. | 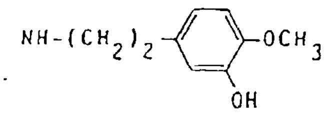 | I | |
| 60. | 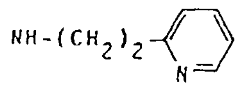 | I | |
| 61. | 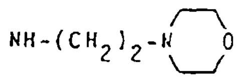 | I | – |
| 62. | 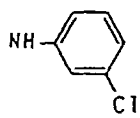 | I | – |
| 63. | 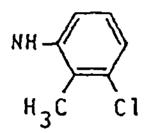 | I | Cl |
| 64. | 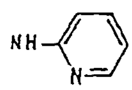 | I | – |
| 65. | 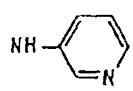 | I | Cl |
| 66. | 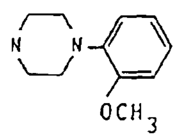 | I | Cl |
| 67. | 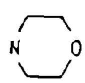 | I | Cl |
| 68. | 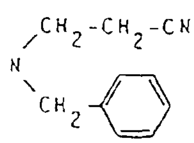 | I | Cl |

Tabelle 4

Strukturtyp:

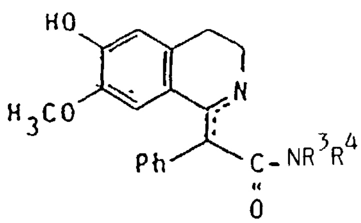

| Nr. | $NR^3R^4$ | Struktur | Salzform |
|---|---|---|---|
| 69. | $N(C_2H_5)_2$ | I | |

Tabelle 5

Strukturtyp:

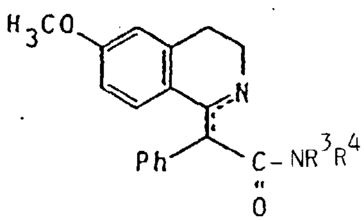

| Nr. | $NR^3R^4$ | Struktur | Salzform |
|---|---|---|---|
| 70. | $NHCH_3$ | I | |
| 71. | $NHC_2H_5$ | I | |
| 72. | 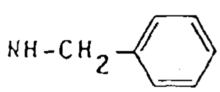 | I | |
| 73. | 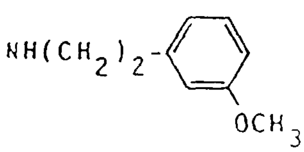 | I | |
| 74. | $N(CH_3)C_2H_5$ | I | |

Tabelle 6

Strukturtyp:

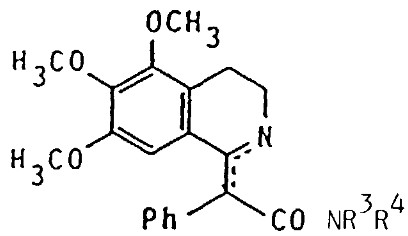

| Nr. | $NR^3R^4$ | Struktur |
|---|---|---|
| 75. | 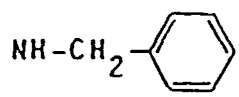 | I |
| 76. | 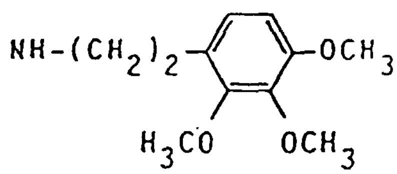 | I |
| 77. | $N(CH_3)C_2H_5$ | I |
| 78. | 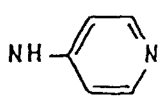 | II |
| 79. | 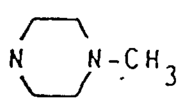 | I |

Tabelle 7

Strukturtyp:

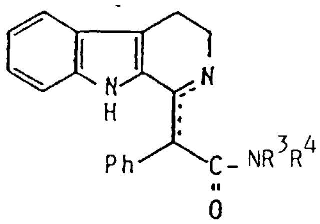

| Nr. | $NR^3R^4$ | Struktur | Salzform |
|---|---|---|---|
| 83. | $NH-(CH_2)_2-$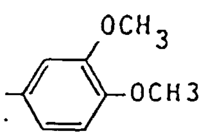 | II | - |
| 84. | $NH-(CH_2)_2-$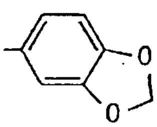 | II | - |
| 85. | $NH-(CH_2)_2-$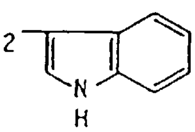 | I<br>II | - |
| 86. | 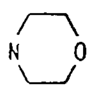 | I | - |
| 87. | $NH-CH_2-CH(CH_3)_2$ | II | - |
| 88. | $NH-(CH_2)_3-N(CH_3)_2$ | II | - |
| 89. | $NH-(CH_2)_2-$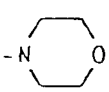 | II | - |

Tabelle 8

Strukturtyp:

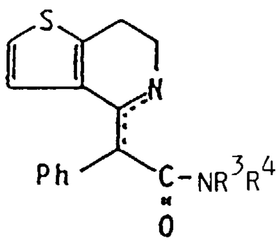

| Nr. | $NR^3R^4$ | Struktur | Salzform |
|---|---|---|---|
| 91. | $NH-(CH_2)_2-$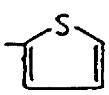 | II | - |

Tabelle 9

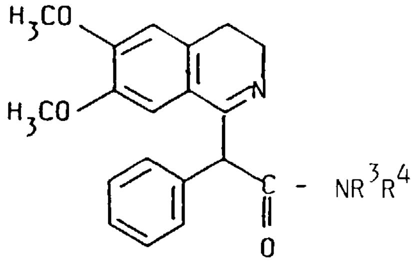

Strukturtyp I

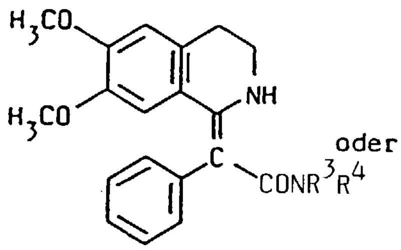

oder

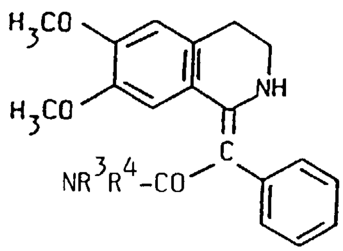

II′ II′′

Strukturtyp II

| Verbindung | $NR^3R^4$ | Strukturtyp | Fp[°C] |
|---|---|---|---|
| A | 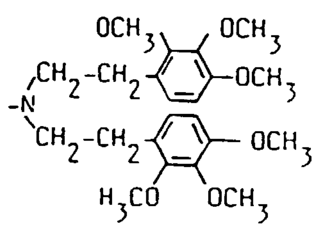 .HCl | I | 56-64 |
| B | 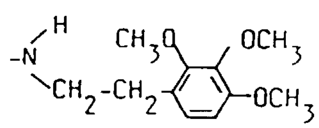 .HCl | I | 176-184 |

| Verbindung (Salzform) | | $NR^3R^4$ | Strukturtyp | Fp[°C] |
|---|---|---|---|---|
| C | | 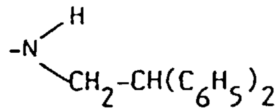 | I | 166-168 |
| D | | 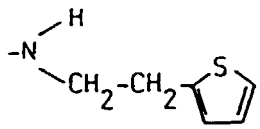 | II | 102-104 |
| E | (Cl) | 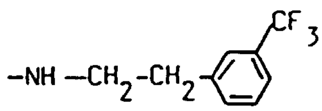 | I | 187 |
| F | (-) | 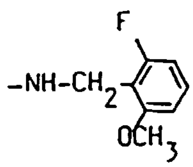 | I | 94-96 |
| G | (Cl) | 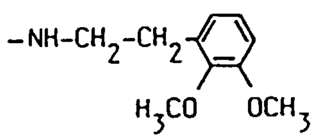 | II | 139-142 |
| H | (-) | 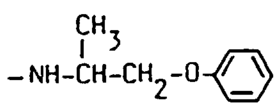 | II | 133-135 |
| J | (-) | 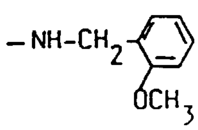 | II | 143-145 |
| K | (-) | 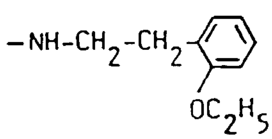 | II | 96-98 |

| Verbindung (Salzform) | $NR^3R^4$ | Strukturtyp | Fp[°C] |
|---|---|---|---|
| L (-) | 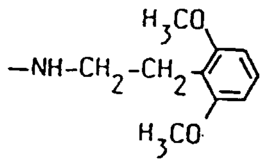 | II | 118-120 |
| M (-) | 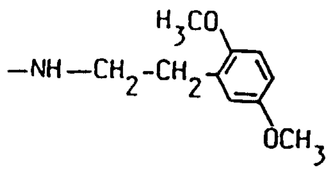 | II | 112-114 |
| N (Cl) | 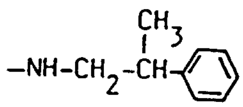 | I | 95-99 |
| O (-) | 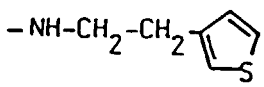 | I | 114-116 |
| P (-) | 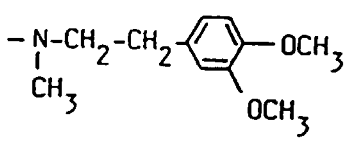 | I | 66-73 |
| Q (Cl) | 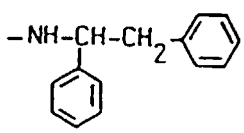 | II | 205-209 |

Tabelle 10

Strukturtyp: 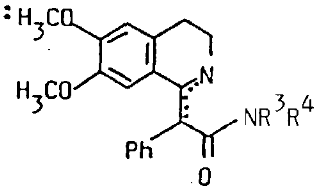

| Nr. | $NR^3R^4$ | Struktur | Salzform | Fp[°C] |
|---|---|---|---|---|
| 101 | $-NH-CH_2-CH_2-CH($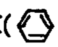$)_2$ | I | Oxalat | 138-140 |
| 102 | $-NH-CH_2-CH_2-$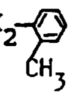 | I | Oxalat | 153-155 |
| 103 | 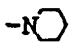 | I | Oxalat | 117-123 |
| 104 | $-NH-CH_2-CH_2-$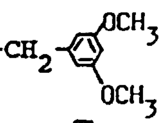 | I | Oxalat | 154-155 |
| 105 | $-NH-CH_2-CH_2-$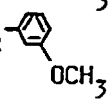 | I | Oxalat | 127-129 |
| 106 | $-NH-CH_2-$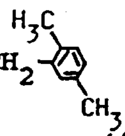 | I | Oxalat | 132-135 |
| 107 | $-NH-CH_2-CH$ (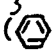$)-CH_3$ | I | Oxalat | 145-147 |
| 108 | $-NH-(CH_2)_3-$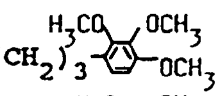 | I | Base | 118-120 |
| 109 | $-NH-CH_2-CH_2-$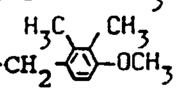 | II | Base | 118-120 |
| 110 | $-N(-CH_2-CH_2-$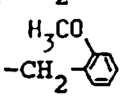$)_2$ | I | Oxalat | 153-156 |
| 111 | $-N[(CH_2)_5CH_3]_2$ | I | Oxalat | 81-84 |
| 112 | $-N(CH_2CH_2CH_3)_2$ | I | Oxalat | 57-59 |
| 113 | $-N[(CH_2)_4CH_3]_2$ | I | Oxalat | ...... |
| 114 | $-NH-CH_2-CH_2-$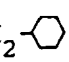 | I | Oxalat | 125-126 |

| Nr. | $NR^3R^4$ | Struktur | Salzform | Fp[°C] |
|---|---|---|---|---|
| 115 | $-NH-CH_2-CH_2-CH_2-$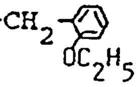 | I | Base | 103-105 |
| 116 | $-N[(CH_2)_3CH_3]_2$ | I | Oxalat | 119-121 |
| 117 | $-N[CH_2CH(CH_3)_2]_2$ | I | Oxalat | 130-131 |
| 118 | -NH-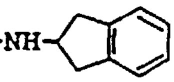 | I | Oxalat | 176-178 |
| 119 | $-NH-CH_2-CH_2-$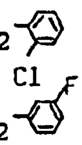 | I | Oxalat | 160-162 (Zers.) |
| 120 | $-NH-CH_2-CH_2-$ | I | Oxalat | 170-172 |
| 121 | $-NH-CH_2-CH_2-$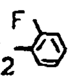 | I | Oxalat | 158-160 (Zers.) |
| 122 | $-NH-CH_2-CH_2-$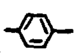- Cl | I | Oxalat | 166-168 |
| 123 | $-NH-(CH_2)_9-CH_3$ | I | Oxalat | 99-101 |
| 124 | $-N(CH_2CH_2-$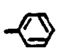$)_2$ | I | Oxalat | 127-143 |
| 125 | $-N(CH_2-CH_2-$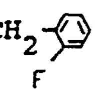$)_2$ | I | Oxalat | 144-149 |
| 126 | $-N-(CH_2-$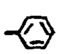$)_2$ | I | Oxalat | 137-139 |
| 127 | $-NH-CH_2CH_2-$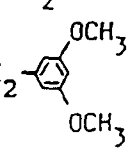 | I | HCl | 185-187 |
| 128 | $-N(CH_2CN)_2$ | I | Base | |
| 129 | -NH-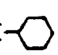 | II | Base | |
| 130 | $-NH-CH_2-$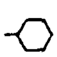 | II | HCl | |
| 131 | -NH-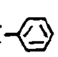 | II | HCl | |

| Nr. | $NR^3R^4$ | Struktur | Salzform | Fp[°C] |
|---|---|---|---|---|
| 132 | $-NH-CH_2-Adam(1)$ | II | HCl | |
| 133 | 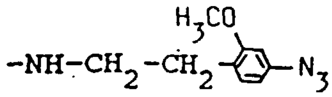 | I | Oxalat | |
| 134 | 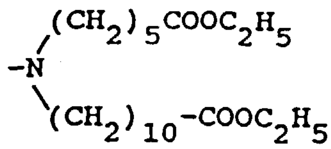 | I | Base | |
| 135 | 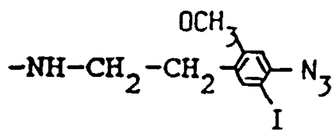 | I | HCl | |
| 136 | 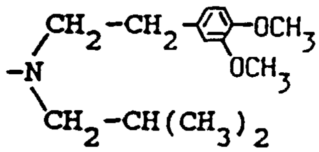 | I | HCl | |
| 137 | 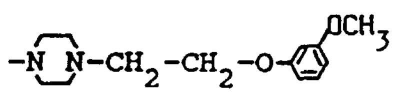 | I | $(Oxalat)_2$ | |
| 138 | 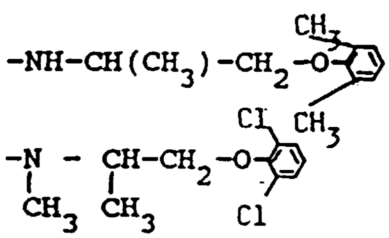 | I | HCl | |
| 139 | | I | HCl | |
| 140 | 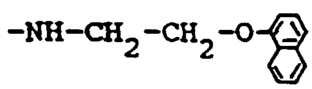 | I | Base | |

| Nr. | $NR^3R^4$ | Struktur | Salzform | Fp[°C] |
|---|---|---|---|---|
| 148 | NH-$CH_2$ 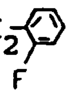 | I | Oxalat | 118-120 |
| 149 | NH-$CH_2$ 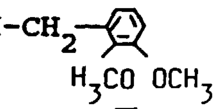 | I | HCL | 195-197 |
| 150 | NH-$CH_2$ 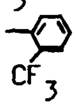 | I | HCL | 140-143 |
| 151 | NH-$CH_2$ 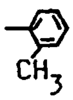 | I | HCL | 193-195 |
| 152 | NH 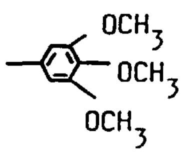 | I | HCL | 155-158 |
| 153 | NH-$CH_2$ 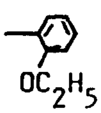 | I | HCL | 168-170 |
| 154 | NH-$CH_2$ 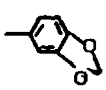 | I | HCL | 172-174 |

Adam = Adamantyl

Tabelle 11

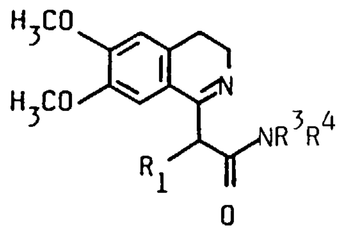

| $R_1$ | $NR^3R^4$ | Salzf. | |
|---|---|---|---|
| 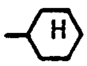 | 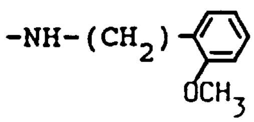 | HCl | Fp. 116-127°C |
| 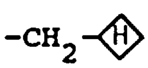 | 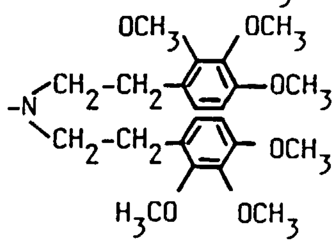 | BS | Rf: 0,39 (Essigester) |

Tabelle 12

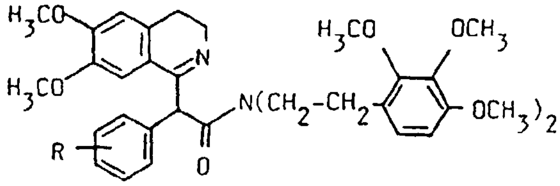

| Nr. | R | Struktur | Salzform | Fp[°C] |
|---|---|---|---|---|
| 1 | 4-$CH_3$ | I | Oxalat | |
| 2 | 4-F | I | Oxalat | |
| 3 | 3-Cl | I | Oxalat | |
| 4 | 4-Br | I | Oxalat | |
| 5 | 2-$OCH_3$ | I | Oxalat | |
| 6 | 3-$OCH_3$ | I | Oxalat | |
| 7 | 4-$OCH_3$ | I | Oxalat | |
| 8 | 3,4-di-$OCH_3$ | I | Oxalat | |
| 9 | 3,4,5-tri-$OCH_3$ | I | Oxalat | |
| 10 | 4-$N_3$ | I | Oxalat | |
| 11 | 3-I,4-$N_3$ | I | Oxalat | |
| 12 | 2-$CH_3$ | I | Oxalat | |
| 13 | 2-Br | I | Base | |
| 14 | 2,4-di-Cl | I | Oxalat | |

| Nr. | R | Struktur | Salzform | Fp[°C] |
|---|---|---|---|---|
| 15 | 3-F | I | Oxalat | |
| 16 | 2-Cl | I | Base | |

Tabelle 13

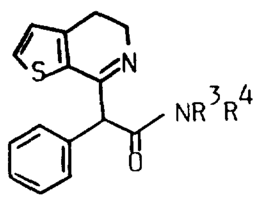

I

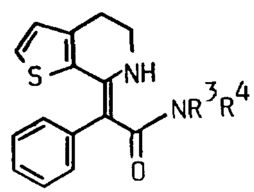

II

| Nr. | $NR^3R^4$ | Salzform | Struktur | Fp(°C) | %Inhib. |
|---|---|---|---|---|---|
| | 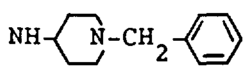 | 1,5 Fu | I | 177-178 | 40,44 |
| | " | BS | II | 158-159 | 0,0 |
| | 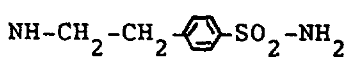 | BS | II | 165-167 | 50,64 |
| | 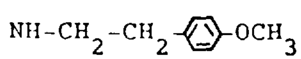 | MS | I | 132-133 | 67,73 |

| Nr. | $NR^3R^4$ | Salzform | Struktur | Fp(°C) | %Inhib. |
|---|---|---|---|---|---|
| | $NH-CH_2-CH_2-$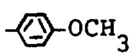 | BS | II | 84-85 | 65,50 |
| | $NH-CH_2-CH_2-$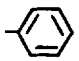 | MS | I | 151-153 | 80,89 |
| | " | BS | II | 110-111 | 39,47 |
| | $NH-CH_2-CH_2-$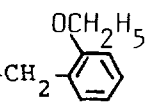 | MS | I | 118-122 | 24,03 |
| | $NH-CH_2-CH_2-$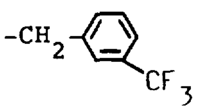 | MS | I | 150-154 | 43,49 |
| | $NH-CH_2-CH_2-$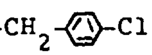 | Cl | I | 144-147 | 15,26 |
| | $NH-CH_2-CH_2-$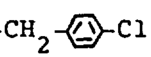 | BS | II | 131-133 | 26,54 |
| | $NH-CH_2-CH_2-$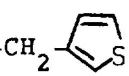 | Cl | I | 114-115 | 0,0 |
| | $NH-CH_2-CH_2-$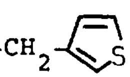 | BS | II | 144-147 | 67,75 |

Tabelle 14

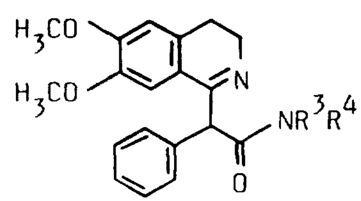

I

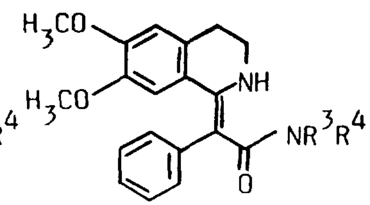

II

| Nr. | $NR^3R^4$ | Salzform | Struktur | Fp(°C) | %Inhib. |
|---|---|---|---|---|---|
| | $NH-CH_2CH_2-$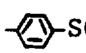$-SO_2NH_2$ | Cl | I | amorph x) | 79,17 |
| | $NHCH_2CH_2CH_2-$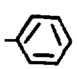 | Cl | I | amorph x) | 73,55 |

| Nr. | $NR^3R^4$ | Salzform | Struktur | Fp(°C) | %Inhib. |
|---|---|---|---|---|---|
| | 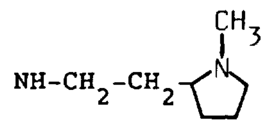 | Cl | I | amorph x) | 60,82 |
| | 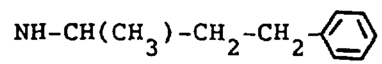 | Cl | I+II | amorph x) | 82,76 |
| | 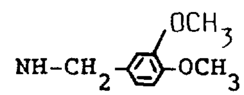 | Cl | I | amorph x) | 50,90 |
| | 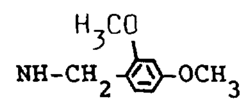 | Ox | I | amorph x) | 56,20 |
| | 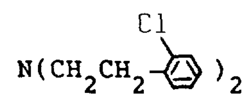 | Ox | I | 142-144 | |
| | 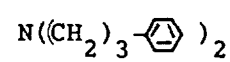 | BS | I | 105-106 | 22,39 |
| | 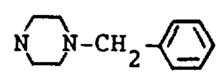 | $Cl_2$ | I | 208-210 | |

| Nr. | $NR^3R^4$ | Salzform | Struktur | Fp(°C) | %Inhib. |
|---|---|---|---|---|---|
| | 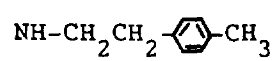 | MS | I | amorph x) | 79,60 |
| | 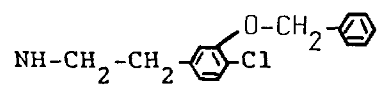 | Cl | I | amorph x) | 36,71 |
| | 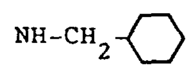 | Cl | I | amorph x) | 30,87 |

x) durch NMR charakterisiert

Tabelle 15

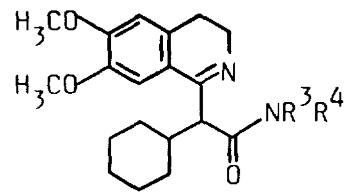

I

| Nr. | $NR^3R^4$ | Salzform | Struktur | Fp(°C) | %Inhib. |
|---|---|---|---|---|---|
| | $NH-CH_2CH_2-$ 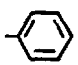 | Cl | I | 134-136 | |
| | $NH-CH_2-CH_2-$ 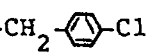 -Cl | Cl | I | 166-168 | |
| | $NH-CH_2-$ 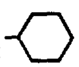 | Cl | I | 142-144 | |
| | $NH-(CH_2)_3-$ 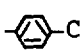 $-C(CH_3)_3$ | BS | I | 119 | 84,43 |

Tabelle 16

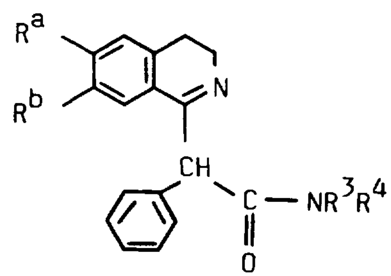

| Nr. | Salzform | $R^a$ | $R^b$ | $NR^3R^4$ | Fp. | %H | $IC_{50}$ |
|---|---|---|---|---|---|---|---|
| | MS | HO- | $CH_3O$- | 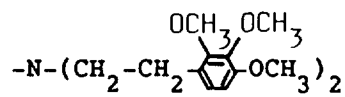 | | 87 | $3{,}47.10^{-6}$ |
| | MS | $CH_3O$- | HO- | " | | 0 | --- |

| Nr. | Salzform | $R^a$ | $R^b$ | $NR^3R^4$ | Fp. | %H | $IC_{50}$ |
|---|---|---|---|---|---|---|---|
| | MS | $CH_3O-$ | $CH_3O-$ | 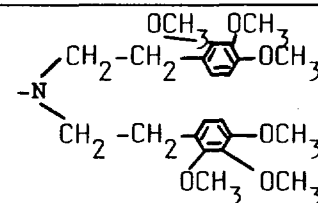 | | | |
| | OX | $CH_3O-$ | H- | $-NH-CH_2-CH_2-$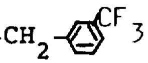 | 60-70 | 30,41 | |
| | MS | 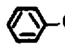$-CH_2-O-$ | H- | " | 182-185 | 14,65 | |
| | OX | HO- | H- | " | 120-130 | | |
| | OX | $CH_3O-$ | $CH_3-$ | " | 143 | 10,47 | |
| | OX | 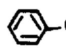$-CH_2O-$ | $CH_3-$ | " | 156-157 | 49,03 | |

| Nr. | Salzform | $R^a$ | $R^b$ | $NR^3R^4$ | Fp. | %H | $IC_{50}$ |
|---|---|---|---|---|---|---|---|
| | OX | HO- | $CH_3$- | " | 80-95 | | |
| | OX | $CH_3O$- | Cl- | " | 143-145 | 44,65 | |
| | OX | 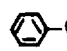-$CH_2O$- | Cl- | " | 130-132 | 32,90 | |
| | OX | HO- | Cl- | " | 182-183 | | |
| | MS | 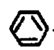-$CH_2$-O- | $CH_3O$- | " | | 31,8 | |
| | MS | $CH_3O$- | 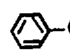-$CH_2O$- | " | | 22,9 | |
| | OX | $CH_3O$- | $CH_3$- | -N ($CH_2$-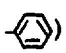)$_2$ | 145-151 | 58,3 | |

| Nr. | Salzform | $R^a$ | $R^b$ | $NR^3R^4$ | Fp. |
|---|---|---|---|---|---|
| | OX | $CH_3O-$ | H- | " | 115-125 |
| | OX | $CH_3O-$ | Cl- | " | 131-135 |
| | OX | 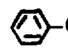$-CH_2-O-$ | Cl- | " | 135-142 |
| | OX | 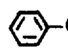$-CH_2-O-$ | $CH_3-$ | " | 162-164 |
| | OX | 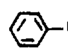$-CH_2-O-$ | H- | " | 145-147 |
| | OX | HO- | Cl- | " | 187-190 |
| | OX | HO- | $CH_3-$ | " | 193-200 |
| | OX | HO- | H- | " | 154-157 |

Tabelle 17

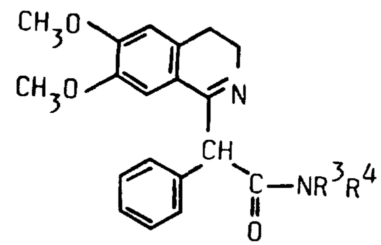

| Nr. | $NR^3R^4$ | Salzform | Fp(°C) | %Hem. | $IC_{50}$ |
|---|---|---|---|---|---|
| | 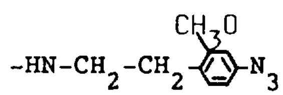 | OX | amorph* | 68,2 | |
| | 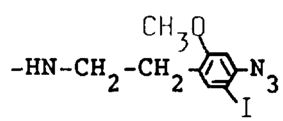 | Cl | amorph* | 36,95 | |

| Nr. | $NR^3R^4$ | Salzform | Fp(°C) | %Hem. | $IC_{50}$ |
|---|---|---|---|---|---|
| | 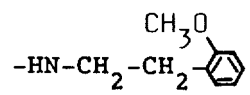 | Cl | | 69,7 | |
| | 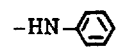 | Cl | amorph* | | |
| | 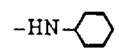 | BS | amorph* | | |
| | 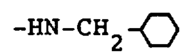 | Cl | amorph* | | |
| | $-HN-CH_2-1-Adam.$ | Cl | amorph* | 65,1 | |
| | 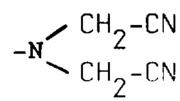 | BS | amorph* | | |
| | 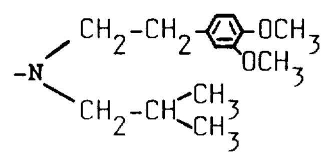 | Cl | amorph* | 12,82 | |

| Nr. | $NR^3R^4$ | Salzform | Fp(°C) | %Hem. | $IC_{50}$ |
|---|---|---|---|---|---|
| | 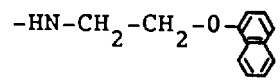 | BS | amorph* | 59 | |
| | 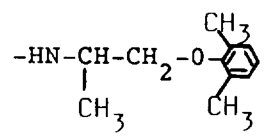 | Cl | amorph* | 72,18 | |
| | 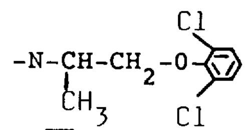 | Cl | amorph* | | |
| | 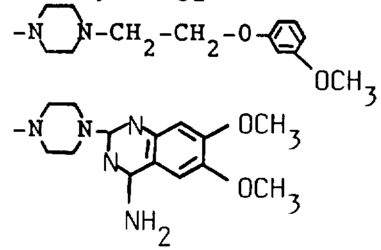 | OX-2 | amorph* | 41,06 | |
| | | BS | amorph* | 7,77 | |

| Nr. | $NR^3R^4$ | Salzform | Fp(°C) | %Hem. | $IC_{50}$ |
|---|---|---|---|---|---|
| | 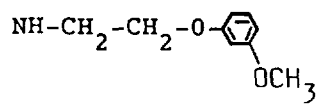 | BS | amorph* | 45,26 | |
| | 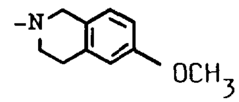 | OX | amorph* | 33,06 | |
| | 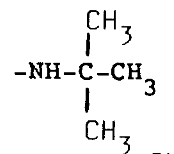 | Cl | 199-201 | 81,60 | |
| | 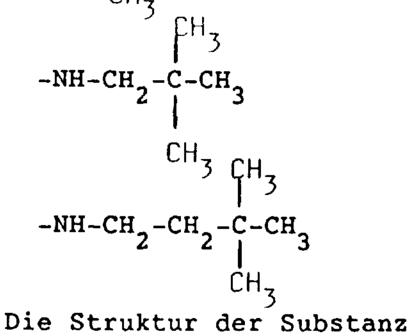 | Cl | amorph* | 52,51 | |
| | | Cl | amorph* | 80,97 | $7,57.10^{-6}$ |

*: Die Struktur der Substanz ist nmr-spektroskopisch chrakterisiert

Tabelle 18

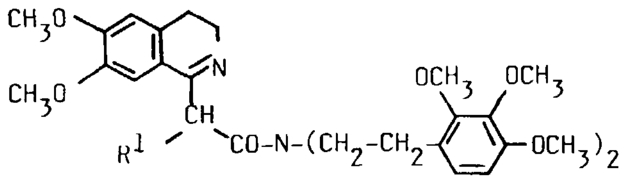

| Nr. | $R^1$ | Salzform | Fp. | %Hem. | $IC_{50}$ |
|---|---|---|---|---|---|
| | 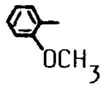 | OX | amorph* | 41,8 | |
| | 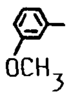 | OX | amorph* | 24,3 | |
| | 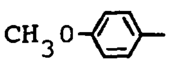 | OX | amorph* | 73,7 | |
| | 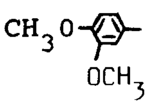 | OX | amorph* | 53,1 | |
| | 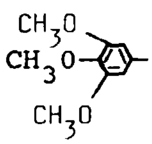 | OX | amorph* | | |
| | 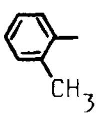 | OX | amorph* | 68,22 | |
| | 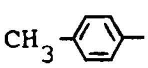 | OX | amorph* | | |
| | 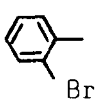 | BS | amorph* | | |
| | 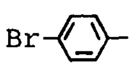 | OX | amorph* | | |

| Nr. | $R^1$ | Salzform | Fp. | %Hem. | $IC_{50}$ |
|---|---|---|---|---|---|
| | 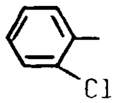 | BS | amorph* | | |
| | 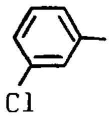 | OX | amorph* | | |
| | 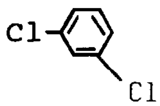 | OX | amorph* | 30,79 | |
| | 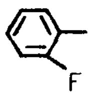 | OX | amorph* | | |
| | 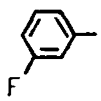 | OX | amorph* | | |
| | 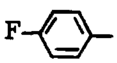 | OX | amorph* | 54,30 | |
| | 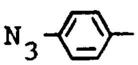 | OX | amorph* | 61,3 | |
| | 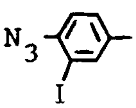 | Cl | amorph* | 5,81 | |
| | 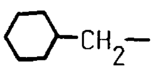 | BS | amorph* | | |
| | 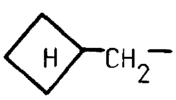 | | amorph* | 56,0 | |
| | 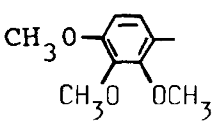 | OX | amorph* | | |
| | 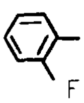 | OX | amorph* | | |

*: Die Struktur der Substanz ist nmr-spektroskopisch charakterisiert.

Tabelle 19

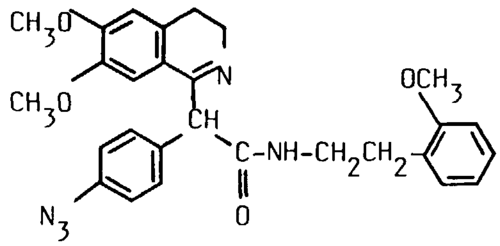

. OX amorph*

% Hem: 50,58

*: Die Struktur der Substanz ist nmr-spektroskopisch charakterisiert.

**Tabelle 20**

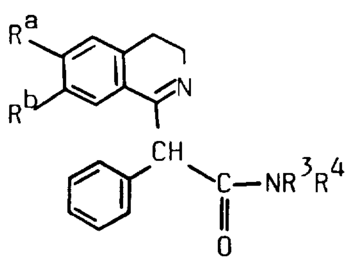

| Nr. | $R^a$ | $R^b$ | $NR^3R^4$ | Salzform | Fp. | %H | $IC_{50}$ |
|---|---|---|---|---|---|---|---|
| | HO- | $CH_3O$- | N(-$CH_2CH_2$-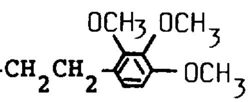 )$_2$ | MS | 163-169 | 86,70 | $3{,}47.10^{-6}$ |
| | Ph-$CH_2$-O- | $CH_3O$- | " | MS | amorph* | 31.78 | |

| Nr. | $R^a$ | $R^b$ | $NR^3R^4$ | Substanz | Fp. | %Hem. | $IC_{50}$ |
|---|---|---|---|---|---|---|---|
| | $Ph-CH_2-O-$ | $CH_3O-$ | $NH-CH_2CH_2-C(CH_3)_3$ | OX | amorph* | 61,85 | |
| | $Ph-CH_2-O-$ | Cl- | $NH-CH_2CH_2-$ 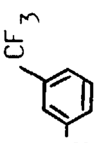 | OX | 130-132 | 32,9 | |
| | $CH_3O-$ | Cl- | " | OX | 143-145 | 44,65 | |
| | $Ph-CH_2-O-$ | $CH_3-$ | " | OX | 156-157 | 49,03 | |

* Die Struktur der Substanz ist nmr-spektroskopisch charakterisiert

Ein weiterer Gegenstand der vorliegenden Erfindung ist die Verwendung der genannten Verbindungen für die Herstellung von Mitteln zur Behandlung chronisch inflammatorischer Prozesse, der Colitis Ulcerosa und des Morbus Crohn und von Mitteln mit antiproliferativer Wirkung. Die Wirkung der Verbindungen kann durch ihre Hemmung unselektiver Kationenkanäle (UKK) erklärt werden.

Der Pathophysiologie des chronischen Bronchialasthma liegen entzündliche Prozesse zugrunde, die durch die Aktivierung inflammatorischer Zellen vermittelt werden. (BARNES, 1987; SEIFERT und SCHULTZ, 1991).

Die rezeptor-regulierte Aktivierung inflammatorischer Zellen (z.B. neutrophile Granulozyten und Mastzellen bzw. deren permanente Zellinien HL-60 Zellen oder sensibilisierte, d.h. Gammaglobulin E beladene RBL-Zellen) wird unabhängig von der Art der stimulierenden Agonisten (z.B. Endothelin, PAF, Leukotriene, chemotaktisches Peptid fMLP oder Antigen gegen sensibilisierte Mastzellen) durch Blocker unselektiver Kationenkanäle (UKK) inhibiert (RINK, 1990). Durch diese Kanäle gelangt extrazelluläres Calcium in die Zellen, das für die Persistenz der rezeptor-vermittelten Zellaktivierungen erforderlich ist (PUTNEY, 1990). Wird diese Calciumzufuhr unterbrochen resultiert eine Blockade der Aktivierung inflammatorischer Zellen.

Klassische Calciumantagonisten vom Dihydropyridin- bzw. Phenylalkylamin-Typ hemmen weder UKKs noch inflammatorische Prozesse (WELLS et al., 1986).

Als Maß der Zellaktivierung bzw. als Maß von deren Hemmung durch UKK-Blocker wird fluorometrisch die Kinetik der cytoplasmatischen Calciumionenkonzentration in Fura-2-beladenen Zellen anhand der von GRYNKIEWICZ et al. (1985) beschriebenen Methode quantifiziert. Diese Vorgehensweise hat sich im Rahmen dieser Erfindung als zuverlässige Screeningmethode zur Auffindung von UKK-Blockern erwiesen.

Zur spezifischen Charakterisierung von Blockern unselektiver Kationenkanäle eignet sich die sogenannte funktionelle THAPSIGARGIN-Inhibition. THAPSIGARGIN ist ein von THASTRUP et al. (Proc. Natl. Acad. Sci. (USA), 87, 2466-2470, 1990) beschriebener Tumorpromotor, der selektiv und irreversibel die $Ca^{2+}$ATPase intrazellulärer, $IP_3$-sensitiver $Ca^{2+}$-Speicher hemmt. Infolge dessen kommt es zu einer raschen Entleerung der $Ca^{2+}$-Speicher. Wie von J. PUTNEY (Calcium, 11, 611-624, 1990) beschrieben stellt die Entleerung dieser Speicher den physiologischen Reiz für die Öffnung unselektiver Kationenkanäle in der Zellmembran dar. Die Folge ist ein massiver Einstrom von $Na^+$ und $Ca^{2+}$ in die Zelle. Aufgrund dieser Eigenschaften eignet sich Thapsigargin als indirekter Stimulator zur agonisten- und $IP_3$-unabhängigen Öffnung unselektiver Kationenkanäle.

Im Rahmen der vorliegenden Erfindung wurde die Thapsigargin-Stimulation unselektiver Kationenkanäle an HL 60 Zellen (menschliche Leukämiezelle), an hippocamplen und corticalen Neuronenzellen sowie an RBL-Zellen (rat basophilic lymphoma) erfolgreich durchgeführt und somit wurde die Existenz dieser Kanäle in den jeweiligen Zellinien nachgewiesen.

Die zytoplasmatische $Ca^{2+}$Konzentration ($[Ca^{2+}]_i$) spielt eine wichtige Rolle bei der Zellproliferation und beim Tumorwachstum (Übersicht siehe L.R. ZACHARSKI, Journal of Medicine 19: 145-177, 1988). Insbesondere der durch Rezeptoraktivierung mit konsekutiver Inositoltrisphosphat-($IP_3$-)-Vermittlung stimulierte $Ca^{2+}$-Einstrom in die Zelle soll von entscheidender Bedeutung sein für die oncogene Zellproliferation (U. KIKKAWA und Y. NISHIZUKA, Ann. REV. CELL. BIOL. 2: 149-178, 1986). Dieser Mechanismus spielt auch eine Rolle bei der Metastasenbildung und der "Multi-Drug-Resistance". (Übersicht siehe die obengenannten Veröffentlichung von L.R. ZACHARSKI.) J. MED. 19: 145-177, 1988.

Diese Hypothese wird dadurch gestützt, daß Thapsigargin als indirekter Stimulator unselektiver Kationenkanäle (UKK) sowohl zu einem $Ca^{2+}$-overload in der Zelle führt als auch ein hochwirksamer Tumorpromotor ist. (V. THASTRUP et al. Proceedings of the NATL. Acad. Sci: (USA) 87: 2466-2470, 1990.)

Die Blockade des $Ca^{2+}$-Einstroms durch die UKK führt zu einer Normalisierung der intrazellulären Ca-Ionenkonzentration und somit zu einer Hemmung des Tumorwachstums etc.

Klassiche Calciumantagonisten hemmen diese UKK nicht. Überraschend ist festgestellt worden, daß die Verbindungen dieser Erfindung den Calciumeinstrom in die Zelle durch die UKK hemmen.

Wie von S. H. MURCH et al. (Lancet 339 : 381-385, 15. Febr. 1992) gezeigt wurde, spielt Endothelin I eine wichtige pathophysiologische Rolle bei entzündlichen Darmerkrankungen wie der Colitis ulcerosa und des Morbus Crohn. Mit Hilfe immunhistochenischer Methoden konnte festgestellt werden, daß Patienten mit M. Crohn im Bereich der Submucosa und Patienten mit Colitis ulcerosa im Bereich der Lamina propria des Dickdarmepithels signifikant und stark erhöhte Endothelin I Konzentrationen im Vergleich zu gesunden Normalpersonen aufweisen. Es wird angenommen, daß die lokale Ausschüttung von Endothelin massive Vasospasmen mit konsekutiver disseminierter Ischämie mit Mikroinfarkten hervorruft, die als eigentliche Ursache der genannten Erkrankungen angesehen werden. Die vasospasmogene Effektivität des Endothelins wird über einen $Ca^{2+}$-overload vaskulärer Myozyten erklärt. Dabei löst Endothelin primär eine $IP_3$-vermittelte intrazelluläre $Ca^{2+}$-Freisetzung aus, an die sich ein massiver transmembranärer $Ca^{2+}$-Einstrom durch dihydropyridin-insensitive Kanäle anschließt. (M. S. Simonson et al. Clin. Invest. Med. 14: 499-507, 1991; T. Masakai, J. Cardiovasc. Pharmacol. 13:Suppl. 5, S1-S4, 1989; D. W. Hay, R. J. Pharmacol. 100: 383-392, 1990) Bei diesen Kanälen handelt es sich um unselektive Kationen Kanäle, deren Existenz kürzlich auch in Zellen der Dickdarmmukosa beschrieben wurde. (Chr. Siemer und H. Gögelein, Europ. J. Physiol. 420: 319-328, 1992).

Als geeignetes Screening Modell zur Auffindung funktioneller Endothelinantagonisten hat sich die Endothelin stimulierte Aktivierung Fura-2 beladener menschlicher Leukämiezellen (HL 60 Zelle) bewährt. In Anlehnung an G. GRYNKIEWICZ et al. (J. Biol. Chem. 260:340-3450, 1985)läßt sich die intrazelluläre $Ca^{2+}$-Konzentration im Cytoplasma von HL 60 Zellen (Suspensionen) spektrofluorometrisch verfolgen und als Maß der Zellaktivierung durch Endothelin quantifizieren. Die Stimulation erfolgte durch Zusatz von 0.1 $\mu$M Endothelin, sie ließ sich dosisabhängig durch die erfindungsgemäßen Substanzen inhibieren.

Der funktionelle Endothelinantagonismus der erfindungsgemäßen Substanzen wird über eine Blockade der unselektiven Kationen Kanäle vermittelt. Deshalb eignet sich auch der Nachweis eines funktionellen Thapsigargin-Antagonismus an RBL-hm1-Zellen als Screening Methode für funktionelle Endothelinantagonisten.

Ausführung der Untersuchung:

Für Screeningzwecke werden in $Ca^{2+}$ freiem Inkubationsmedium Fura-2-beladene adhäsive RBL-hm 1-Zellen mit 0,1 µM Thapsigargin stimuliert. Nach 4 Minuten wird extrazelluläres $Ca^{2+}$ auf 1,5 mM restituiert und anhand der Fura-2-Fluoreszenz der exzessive Anstieg der cytoplasmatischen $Ca^{2+}$-Konzentration infolge eines massiven transmembranären $Ca^{2+}$-Einstroms durch unselektive Kationenkanäle registriert.

Dieser Einstrom ist ausschließlich und dosisabhängig zu inhibieren durch unselektive Kationen-Kanal-Blocker. Weder klassische Kalziumantagonisten noch spezifische Blocker von Agonisten, die den $IP_3$-Turnover stimulieren können den durch Thapsigargin indirekt ausgelösten transmembranären $Ca^{2+}$-Einstrom hemmen. Die Verbindungen der vorliegenden Erfindung zeichnen sich durch eine Hemmung der UKK aus.

Die fluorometrische Calciummessung im Cytoplasma einzelner adhärenter RBL-hm1-Zellen erfolgt in Analogie zu der von KUDO und OGURA (1986) für neuronale Zellen beschriebenen Methode. Verwendet wird ein AXIOVERT 35 Fluoreszenzmikroskop von ZEISS in Kombination mit einem Imaging-System von HAMAMATSU, bestehend aus dem ICMS-Bildverarbeitungssystem, Restlichtkamera mit Kontrolleinheit und Bildverstärker DVS 3000.

Die Kinetik der cytoplasmatischen $Ca^{+2}$-Konzentration wird als Konzentrations-Zeit-Kurve nach der durch Thapsigargin (0,1 µM) stimulierten Zellaktivierung fortlaufend aufgezeichnet. Verglichen werden die Kurven zweier aktivierter Zellkulturen in Gegenwart und Abwesenheit von 10 µM Testsubstanz. Die Fläche unter diesen Kurven (area under the curve = AUC) wird integriert und als Maß der Zellaktivierung registriert. Die inhibitorische Wirkungsstärke der getesteten UKK-Blocker wird ermittelt anhand folgender Beziehung:

$$\%H = 100 - \frac{AUC_{inh} \times 100}{AUC_{(Kontrolle)}}$$

%H = die prozentuale Hemmung des Calciumeinstroms durch unselektive Kationenkanäle der stimuliert und durch 10 µM Testsubstanz inhibiert wird.

$AUC_{inh}$ = Fläche unter der Kurve, die in Gegenwart des Stimulans plus 10 µM inhibitorischer Testsubstanz aufgezeichnet wird.

AUC Kontr. = Fläche unter der Kurve, die nur nach Zusatz des Stimulans registriert wird.

Literatur zu den obigen Erläuterungen:

BARNES P.J., I.W. RODGER und N.C. THOMSON
Pathogenesis of asthma, in "ASTHMA, basic mechanisms and clinical management"
ED by P.J. BARNES; ACADEMIC PRESS, LONDON, 1988

GRYNKIEWICZ G., M. POENIE und R.Y. TSIEN
A new generation of $Ca^{2+}$-indicators with greatly improved fluorescence properties
J. BIOL. CHEM. 260: 3440-3450, 1985

HIDE, M. und M.A. BEAVEN
Calcium influx in a rat mast cell (RBL-2H3) line J. BIOL. CHEM. 266 15221-15229, 1991

KUDO, Y. und A. OGURA
Glutamate-induced increase in intracellular $Ca^{2+}$-concentration in isolated hippocampal neurones
BR. J. PHARMACOL. 89: 191-198, 1986

PUTNEY, J.W., jr.
Capacitative Calcium entry revised
CELL CALCIUM 11: 611-624, 1990

RINK, T.J.
Receptor-mediated calcium entry
FEBS LETT. 268: 381-385, 1990

SEIFERT, R. und G. SCHULTZ
The superoxide forming NADPH oxidase of phagocytes: An enzym system regulated by multiple mechanism
REV. PHYSIOL. BIOCHEM. PHARMACOL., Vol. 117, SPRINGER VERL., 1991

WELLS, E., C.G. JACKSON, S.T. HARPER, J. MANN and R.P. EAOY
Characterization of primate bronchoalveolar mast cells II, inhibition of histamine, $LTC_4$ and $PGF_{2\alpha}$ release from primate bronchoalveolar mast cells and a comparison with rat peritoneal mast cells J. IMMUNOL. 137: 3941-3945, 1986.

Meßergebnisse:

Angegeben wird die prozentuale Hemmung der UKK nach Thapsigarginstimulation (0,1 μM Thapsigargin) in RBL-hm 1 Zellen. Die einheitliche Konzentration der Testsubstanzen ist $10^{-5}$ Mol).

Tabelle 21 (Angaben zu Verbindungen der Tabellen 1-10)

RBL - hm 1 Zellen - Thapsigargin (0,1 µM)-Stimulation

| Verbindung Nr. | % Hem. |
|---|---|
| 3 | F 41.91 |
| 4 | F 66.53 |
| 5 | F 80.16 |
| 9 | F 67.59 |
| 14 | F 80.16 |
| 17 | F 52.53 |
| 18 | F 57.53 |
| 20 | F 57.38 |
| 33 | 60.55 |
| 36 | F 52.19 |
| 37 | F 75.77 |
| 38a (oxalat) | 52.64 |
| 39 | 48.62 |
| 40 | 64.99 |
| 41 (Struktur I) | F 57.22 |

| Verbindung Nr. | % Hem. |
|---|---|
| 42 | 84.83 |
| 43 | F 48.69 |
| 46 | 68.06 |
| 48 | 61.34 |
| 83 | 66.76 |
| 89 | 53.38 |
| B | 72.35 |
| C | 63.45 |
| E | F 81.89 |
| F | 48.01 |
| G | 57.52 |
| H | 57.06 |
| K | 76.31 |
| L | 53.14 |
| O | 63.32 |

| Verbindung Nr. | % Hem. |
|---|---|
| Q | 64.28 |
| 101 | 59.62 |
| 102 | F 80.77 |
| 104 | 54.67 |
| 105 | 45.09 |
| 106 | F 65.69 |
| 108 | 44.45 |
| 109 | 74.18 |
| 115 | 75.53 |
| 132 | F 65.14 |
| 133 | 68.15 |

Wirkungsangaben (% Hemmung und $IC_{50}$) für weitere Verbindungen sind in den Tabellen 13-20 enthalten.

Anhand des folgenden Tests kann die funktionelle antiinflammatorische Effektivität gezeigt:

Verwendet werden einzelne an Glasplättchen adhärente RBL-2H3-Zellen (eine den Mastzellen verwandte Tumorzellinie).

Die Kultivierung und Anhaftung der RBL-2H3-Zellen erfolgt in der HIDE und BEAVEN (1991) beschriebenen Methode. Zur Sensibilisierung der adhäsiven RBL-2H3-Zellen werden die Zellen 2 Stunden bei Raumtemperatur mit einer 1:2000 verdünnten käuflichen Gammaglobulin E-Lösung gegen einen Dinitrophenol-Rinderserumalbumin-Komplex (DNP-BSA-Antigen) inkubiert. Anschließend werden die Zellen gewaschen. Durch Zusatz von 0,1 ml DNP-BSA-Lösung (10 µg/ml) erfolgt eine massive immunologische Zellaktivierung, die durch einen cytoplasmatischen $Ca^{2+}$-overload vermittelt wird. Die fluorometrische Calciummessung im Cytoplasma einzelner adhärenter RBL-2H3-Zellen erfolgt

in Analogie zu der von KUDO und OGURA (1986) für neuronale Zellen beschriebenen Methode, die auch weiter oben in dieser Beschreibung erläutert wird.

Als Vergleichssubstanz dient in diesen Untersuchungen (10 µM) Chromoglycat, das eine ca. 50 %ige Hemmung der antigen-induzierten Zellaktivierung bewirkt.

In diesem Test zeigen die oben genannten Verbindungen %H Werte, die den oben angegebenen Werten vergleichbar sind.

Untersuchungen an Mikrokulturen verschiedener menschlicher Tumorzellinien mit Hilfe des Tetrazolium Assays zur Feststellung des antiproliferativen Effektes der erfindungsgemäßen Substanzen zeigte sich überraschenderweise, daß die geprüfte Verbindung 5 bis 100 mal wirkungsstärker als die Vergleichssubstanz Verapamil sind.

Die antiproliferative Effektivität der Testsubstanzen wurde mit Hilfe des von MOSMANN (J. IMMUNOL. METH. 65: 55-63, 1983), DENIZOT et al. (J. IMMUNOL. METH. 89: 271-277, 1986) und von J. ELIASON et al. (INT. J. CANCER 46: 113-117, 1990) beschriebenen MTT-Tests bestimmt. (MTT = (3-(4,5-dimethylthiazol-2yl)2,5-diphenyltetrazoliumbromid] von CHEMICON Inc. El Segundo, Ca, USA). Dieser Indikator wird nur von lebenden Zellen mit intakten Mitochondrien zu einem blauen Formazan Produkt metabolisiert. Folgende menschliche Tumorzellinien wurden in unserem Test verwendet: A 549 (Adenocarcinom der Lunge), A 431 (Epidermiscarcinom der Vulva), PC 3 (Adenocarcinom der Prostata), SK BR 3 (Adenocarcinom der Mamma), HT 29 (CX1 1) (Adenocarcinom des Colon) und K 562 (chronisch-myeloische Leukämiezelle). Der Test wurde auf Mikrotiterplatten durchgeführt. Jedes Weil enthielt 100 µl einer Zellsuspension (0,2 x $10^6$ Zellen/ml). Als Inkubationsmedium diente RPMI 1640 mit 10% Hitze-inaktiviertem fetalen Kälberserum und 50 µg/ml Gentamycin. Die Zeilsuspensionen wurden 0, 24, 48 oder 72 Stunden bei gesättigter Luftfeuchtigkeit in einem $CO_2$ (5%) - Luft (95%)-Gemisch bei 37°C inkubiert in Gegenwart und Abwesenheit von variablen Konzentrationen antiproliferativer Testsubstanzen. Die Testsubstanzen wurden in DMSO (Endverdünnung: 0,1 %) gelöst. Anschließend wurden 10 µl MTT-Lösung (3 mg/ml) und nach 3 Stunden 100 µl einer 0,08 N HCl enthaltenden Isopropanollösung zugesetzt. Nach einer weiteren Stunde wurde die Lichtabsorption bei 570 nm (Vergleichswellenlänge 630 nm) in einem Mikroplattenleser ermittelt. Die Lichtabsorption ist direkt proportional zur Anzahl lebender Zellen. Die haibmaximalen Hemmkonzentrationen der untersuchten Substanzen lagen bei 1 µg/ml.

Die vasospasmolytische Effektivität der obengenannten funktionellen Endothelin- bzw. Thapsigargin-Antagonisten wurde am isolierten Gefäßpräparat bestätigt: An retrograd perfundierten spontan schlagenden LANGENDORFF Herzen aus Ratten wurde die coronare Perfusion fortlaufend mittels elektromagnetischer Flußmessung (Apparatur von Hugo Sachs Elektronik, MARCH), quantifiziert. Mit dieser Meßanordnung lassen sich Ausmaß, Dauer und Verlaufsform von Gefäßspasmen mit großer Genauigkeit registrieren. Perfundiert man mit 100 nM Endothelinkonzentration, so wird der coronare Perfusionsfluß von 11 auf 5 ml/min reduziert. Die Perfusionseinschränkung kann durch die erfindungsgemäßen Substanzen aufgehoben werden. Die Wirkungsstärken der erfindungsgemäßen Verbindungen bezüglich der Thapsigargininhibition an Fura-2-beladenen RBL-hm1-Zellen bzw. der Effektivität der Endothelininhibition an Fura-2 beladenen HL 60 Zellen korrelierte eindeutig mit der am Langendorffpräparat nachgewiesenen vasospasmolytischen Effektivität der untersuchten Substanzen. Es kann daraus geschlossen werden, daß dem vasospasmolytischen Endothelinantagonismus der untersuchten Substanzen eine Blockade der unselektiven Kationen Kanäle unterliegt.

Pharmazeutische Anwendungsbeispiele

| a) Dragees | |
|---|---|
| 1 Drageekern enthält: | |
| Wirkstoff der allgemeinen Formel I | 30,0 mg |
| Milchzucker | 100,0 mg |
| Maisstärke | 75,0 mg |
| Gelatine | 3,0 mg |
| Magnesiumstearat | 2,0 mg |
| | 210,0 mg |

Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässerigen Suspension von Zucker, Titandioxyd, Talkum und Gummi arabicum aufgebracht wird. Die fertigen Dragees werden mit Bienenwachs poliert.

| b) Tabletten | |
|---|---|
| Wirkstoff der allgemeinen Formel I | 30,0 mg |
| Milchzucker | 100,0 mg |
| Maisstärke | 70,0 mg |
| löslich Stärke | 7,0 mg |
| Magnesiumstearat | 3,0 mg |
| | 210,0 mg |

Herstellung

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 210 mg Gewicht verpreßt.

| c) Kapseln | |
|---|---|
| Wirkstoff gemäß Formel I | 20,0 mg |
| Milchzucker | 230,0 mg |
| Maisstärke | 40,0 mg |
| Talk | 10,0 mg |
| | 300,0 mg |

Herstelling

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

| d) Tabletten | |
|---|---|
| Wirkstoff gemäß Erfindung | 40,0 mg |
| Milchzucker | 100,0 mg |
| Maisstärke | 50,0 mg |
| kolloidale Kieselsäure | 2,0 mg |
| Magnesiumstearat | 3,0 mg |
| insgesamt | 200,0 mg |

Herstellung:

Der Wirkstoff wird mit einem Teil der Hilfsstoffe gemischt und mit einer Lösung der löslichen Stärke in Wasser granuliert. Nach dein Trocknen des Granulats wird der Rest der Hilfsstoffe zugemischt und die Mischung zu Tabletten verpreßt.

| e) Dragées | |
|---|---|
| Wirkstoff gemäß Erfindung | 20,0 mg |
| Milchzucker | 100,0 mg |
| Maisstärke | 65,0 mg |
| kolloidale Kieselsäure | 2,0 mg |
| lösliche Stärke | 5,0 mg |
| Megnesiumstearat | 3.0 mg |
| insgesamt | 195,0 mg |

Herstellung:

Der Wirkstoff und die Hilfsstoffe werden, wie in Beispiel 1 beschrieben, zu Tablettenkernen verpreßt, die mit Zucker, Talcum und Gummi arabicum in üblicher Weise dragiert werden.

| f) Suppositorien | |
|---|---|
| Wirkstoff gemäß Erfindung | 50,0 mg |
| Milchzucker | 250,0 mg |
| Suppositorienmasse q.s. ad | 1,7 g |

Herstellung:

Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

| g) Ampullen | |
|---|---|
| Wirkstoff gemäß Erfindung | 20,0 mg |
| Natriumchlorid | 5,0 mg |
| Bi-destilliertes Wasser q.s. ad | 2,0 ml |

Herstellung:

Der Wirkstoff und das Natriumchlorid werden in bi-destilliertem Wasser gelöst und die Lösung in Ampullen steril abgefüllt.

| h) Ampullen | |
|---|---|
| Wirkstoff gemäß Erfindung | 10,0 mg |
| Natriumchlorid | 7,0 mg |
| Bi-destilliertes Wasser q.s. ad | 1,0 mg |

| i) Tropfen | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| Entmineralisiertes Wasser q.s. ad | 100,00 ml |

Herstellung:

Der Wirkstoff und die Konservierungsmittel werden in entmineralsiertem Wasser gelöst, die Lösung filtriert und in Flaschen zu je 100 ml abgefüllt.

**Patentansprüche**

1. Neue Verbindung der allgemeinen Formel I oder deren Tautomeren der allgemeinen Formel II

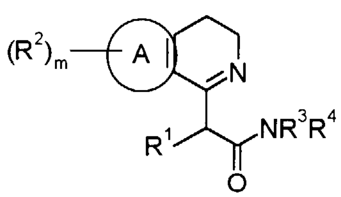

I

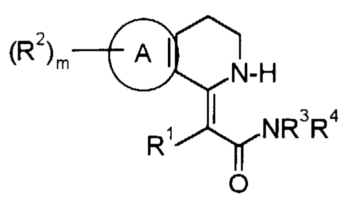

II

worin

A einen Benzo, Indolo oder Thienorest bedeutet;
worin, wenn A Benzo ist, m 2 oder 3 ist, und die Substituenten $R^2$
unabhängig voneinander Hydroxy, $(C_1-C_4)$Alkoxy, Benzyloxy, Halogen (F, Cl, Br, J), $(C_1-C_4)$Alkyl, Methansulfonyloxy oder Methansulfonamido, oder zwei benachbarte Substituenten $R^2$ zusammen $-0-CH_2-0-$ oder $-0-CH_2-CH_2-0-$ sein können; und wenn A Indolo oder Thieno ist, m Null ist;

$R_1$ $(C_4-C_6)$Cycloalkyl, $(C_4-C_6)$Cycloalkyl$(C_1-C_5)$alkyl oder

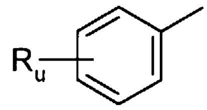

bedeutet,

$R^3$ und $R^4$ unabhängig voneinander

(a) Wasserstoff,

(b) verzweigtes oder unverzweigtes Alkenyl mit 3bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeuten, wobei das Alkyl substituiert sein kann durch
Hydroxy,
$(C_1$-$C_4)$Alkoxy,
Di $(C_1$-$C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,
Cyclohexyl,
Phenoxy,
Naphthyloxy oder Phenyl, wobei dieses Phenyl oder das in der Phenoxygruppe enthaltene Phenyl ein-, zwei- oder dreifach durch Hydroxy, $(C_1$-$C_4)$Alkoxy, Benzyloxy, Halogen (F, Cl, Br, J), $CF_3$, $N_3$, $(C_1$-$C_4)$Alkyl, Aamantyl, $-SO_2NH_2$, $-NHCOCH_3$, $-NHSO_2CH_3$ oder $CH_3SO_2O$- oder durch die Brücke $-O-CH_2-O-$ substituiert sein kann; oder $R^3$ Wasserstoff bedeutet und $R^4$ Cyclohexyl, Phenyl, Fluorophenyl, Pyridyl oder
N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl, die Gruppe

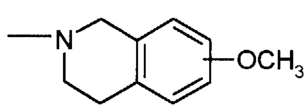

oder
Piperazinyl bedeuten, wobei der Piperazinylring
gegebenenfalls durch Methyl, unsubstituiertes Phenyl, Mono- oder Di $(C_1$-$C_4)$ alkoxyphenyl, Pyrimidinyl, Phenyl $(C_1$-$C_4)$ alkyl oder

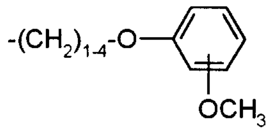

N-substituiert sein kann;

R für $C_1$-$C_4$-Alkyl, Hydroxy, -$N_3$, Halogen (F, Cl, Br, I), $CF_3$, $C_1$-$C_4$-Alkoxy oder -COH steht und

u für 1, 2 oder 3;
oder deren Salze mit physiologisch verträglichen Säuren, Basen oder Komplexbildnern.

2. Verbindung nach Anspruch 1, worin A Benzo ist, m 2 ist und die beiden $R^2$ in den Positionen 6 und 7 sind.

3. Verbindung nach Anspruch 2, worin die beiden $R^2$ unabhängig voneinander Methoxy, Hydroxy, Benzyloxy, Methyl oder Chlor oder zusammen -$OCH_2O$- sind.

4. Verbindung nach Anspruch 3, worin $R^2$ Methoxy, Hydroxy, Benzyloxy oder Methyl ist.

5. Verbindung nach Anspruch 4, worin die beiden $R^2$ Hydroxy oder Methoxy sind.

6. Verbindung nach Anspruch 5, worin die beiden $R^2$ Methoxy sind.

7. Verbindung nach einem der Ansprüche 1-6, worin $R^1$ ($C_4$-$C_6$) Cycloalkyl oder ($C_4$-$C_6$)Cycloalkyl($C_1$-$C_5$)alkyl ist.

8. Verbindung nach Anspruch 7, worin $R^1$ Cylcohexyl, Cyclohexylmethylen oder Cyclobutylmethylen ist.

9. Verbindung nach Anspruch 8, worin $R^1$ Cylcohexyl ist.

10. Verbindung nach einem der Ansprüche 1-6, worin $R^1$

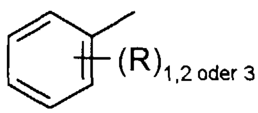

ist.

11. Verbindung I nach einem der Ansprüche 1 bis 10, worin $NR^3R^4$ eine der folgenden Bedeutungen hat

a) in $NR^3R^4$ ist $R^3$ Wasserstoff und $R^4$ $C_2$-$C_6$ Alkyl;
b) in $NR^3R^4$ ist $R^3$ Wasserstoff und $R^4$ verzweigtes oder unverzweigtes Alkinyl mit 3 bis 6 (vorzugsweise 3) Kohlenstoffatomen
c) in $NR^3R^4$ ist $R^3$ Wasserstoff und $R^4$ verzweigtes oder unverzweigtes Alkyl mit 1-4 (vorzugsweise 1 bis 3, insbesondere 2) Kohlenstoffatomen, wobei das Alkyl substituiert ist durch
Methoxy,
Dimethylamino,
Pyrrolidinyl, N-Methylpyrrolidinyl,
Morpholino,
Thienyl,
Adamantyl,
Pyridyl,
N-Benzylpiperidyl,
Cyclohexyl,

Phenoxy,
Naphthyloxy oder 1 oder 2 Phenyl, wobei dieses Phenyl (wenn nur eine Phenylgruppe vorliegt) oder das in der Phenoxygruppe enthaltene Phenyl ein-, zwei- oder dreifach durch Methoxy, Ethoxy, Benzyloxy, Halogen (insbesondere Cl, J), oder $CF_3$, $N_3$, Methyl, tert. Butyl, $-SO_2NH_2$, oder durch die Brücke $-O-CH_2-O-$ substituiert sein kann;
oder $R^3$ Wasserstoff bedeutet und $R^4$ Cyclohexyl, Phenyl, Fluorophenyl, Pyridyl oder N-Benzylpiperidyl;

d) in $NR^3R^4$ sind $R^3$ und $R^4$ unabhängig voneinander Methyl, Ethyl, Benzyl oder

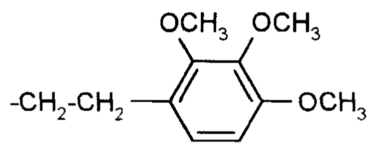

e) $R^3$ und $R^4$ gemeinsam mit dem Stickstoffatom, an das sie gebunden sind, sind Piperidinyl, Morpholinyl, Thiomorpholinyl,
oder
Piperazinyl, wobei der Piperazinylring gegebenenfalls durch Methyl oder Benzyl N-substituiert sein kann;

**12.** Verbindung nach Anspruch 11,
worin $NR^3R^4$ eine der folgenden Bedeutungen hat

a) in $NR^3R^4$ ist $R^3$ Wasserstoff und $R^4$ $C_2$-$C_6$ Alkyl;

b) in $NR^3R^4$ ist $R^3$ Wasserstoff und $R^4$ ist $CH_2CCH$;

c) in $NR^3R^4$ ist $R^3$ Wasserstoff und $R^4$ verzweigtes oder unverzweigtes Alkyl mit 2-4 Kohlenstoffatomen, wobei das Alkyl substituiert ist durch
Methoxy,
Dimethylamino,
N-Methylpyrrolidinyl,
Thienyl,
Adamantyl,
Phenoxy,
Naphthyloxy oder 1 oder 2 Phenyl, wobei dieses Phenyl (wenn nur eine Phenylgruppe vorliegt) oder das in der Phenoxygruppe enthaltene Phenyl ein-, zwei- oder dreifach durch Methoxy, Ethoxy, $N_3$, Methyl, tert. Butyl oder $-SO_2NH_2$ substituiert sein kann;

d) in $NR^3R^4$ sind $R^3$ und $R^4$ unabhängig voneinander Methyl, Ethyl oder

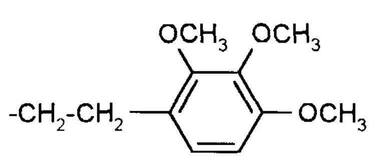

e) $R^3$ und $R^4$ gemeinsam mit dem Stickstoffatom, an das sie gebunden sind, sind Piperazinyl, N-subsitutiert durch Methyl oder Benzyl.

**13.** Verbindung nach Anspruch 11,
worin $NR^3R^4$ eine der folgenden Bedeutungen hat:

a) in $NR^3R^4$ ist $R^3$ Wasserstoff und $R^4$ ist Ethyl, tert. Butyl oder $(CH_2)_1$ oder $_2$-$C(CH_3)_3$;

b) $NR^3R^4$ ist $NHCH_2CCH$;

f) in $NR^3R^4$ ist $R^3$ Wasserstoff und $R^4$ ist Ethyl, Propyl oder Methylpropyl, das durch Phenyl substituiert ist, das ein-, zwei- oder dreifach durch Methyl oder Methoxy oder einfach durch tert. Butyl substituiert ist;

d) in $NR^3R^4$ sind $R^3$ und $R^4$

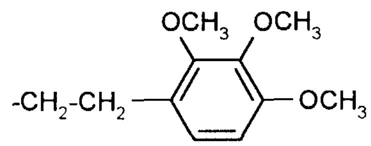

e) $NR^3R^4$ ist

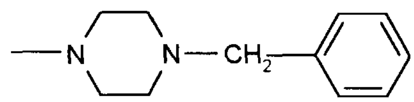

**14.** Verbindung nach Anspruch 11, worin $R^3$ Wasserstoff oder $(C_1$-$C_4)$Alkyl-Phenyl ist und $R^4$ $(C_1$-$C_4)$ Alkyl-Phenyl, wobei in diesen Gruppen jeweils vorzugsweise $C_1$-Alkyl enthalten ist und Phenyl monosubstituiert ist durch Halogen (vorzugsweise Cl oder F), $CF_3$, Methoxy oder Ethoxy, wobei dieser Substituent vorzugsweise in o-Stellung ist.

**15.** Verbindung nach einem der Ansprüche 7 bis 9, worin $NR^3R^4$

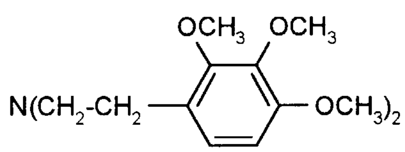

ist.

**16.** Verbindung nach Anspruch 15:

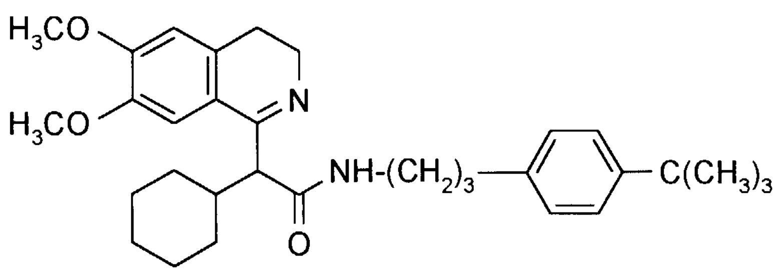

**17.** Verbindung nach Anspruch 10, worin $NR^3R^4$

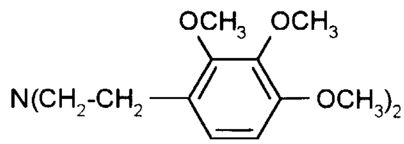

oder

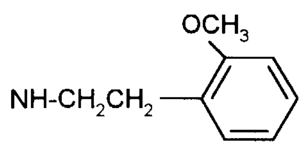

ist.

**18.** Verbindung nach Anspruch 10 oder 17, worin $R^1$ Phenyl ist, das durch Methoxy, Methyl, F oder $N_3$ substituiert ist.

**19.** Verbindung der allgemeinen Formel I der Formel

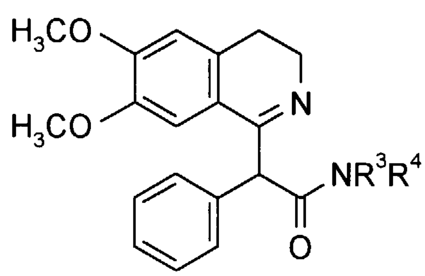

worin $NR^3R^4$ eine der folgenden Bedeutungen hat

| | Struktur |
|---|---|
| 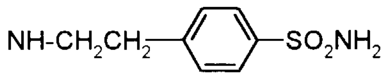 | I |
| 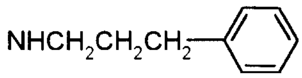 | I |
| 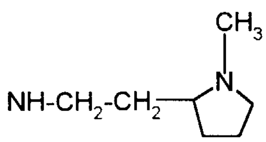 | I |
| 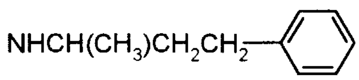 | I+II |
| 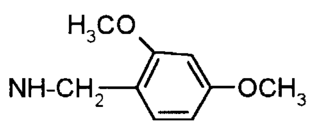 | I |
| 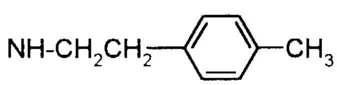 | I |

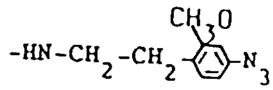

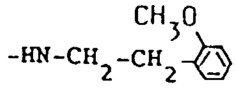

$-HN-CH_2-1-Adam.$

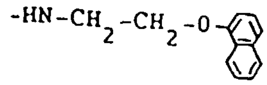

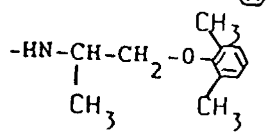

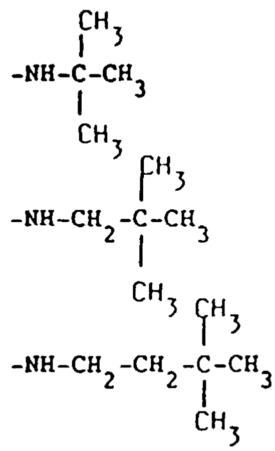

| | Struktur |
|---|---|
| 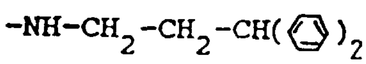 | I |
| 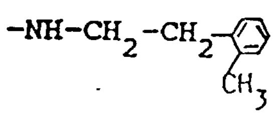 | I |
| 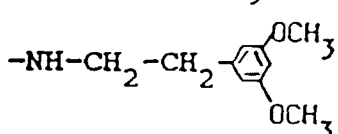 | I |
| 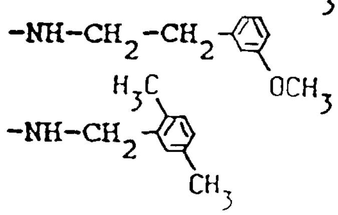 | I<br>I |

Struktur

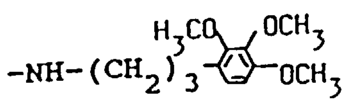

I

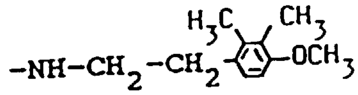

II

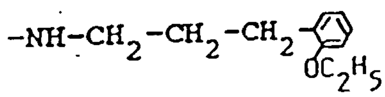

I

$-NH-CH_2-Adam(1)$

II

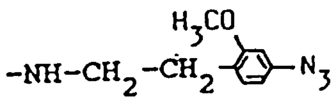

I

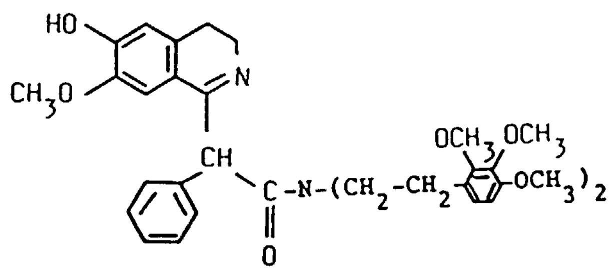

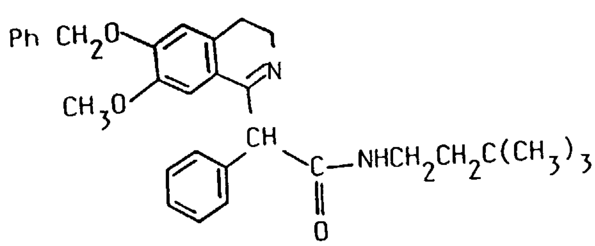

**20.** Verfahren zur Herstellung einer Verbindung nach einem der Ansprüche 1 bis 19, dadurch gekennzeichnet, daß man ein Malonsäurediamid der allgemeinen Formel IV

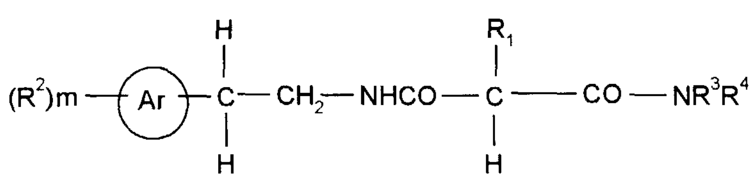

in der $R^1$, $R^2$, $R^3$, $R^4$ und m wie in einem der Ansprüche 1 bis 19 definiert sind und Ar Phenyl, Indolyl oder 2- oder 3-Thienyl bedeutet, in Gegenwart eines Kondensationsmittels cyclisiert und gewünschtenfalls in ihr Salz überführt.

21. Pharmazeutische Zubereitung enthaltend eine Verbindung gemäß einem der Ansprüche 1 bis 19.

22. Verwendung einer Verbindung nach einem der Ansprüche 1 bis 19 zur Herstellung von cardioprotektiven und hirnprotektiven Mitteln, von Mitteln zur Behandlung chronisch inflammatorischer Prozesse, der Colitis Ulcerosa und des Morbus Crohn, und von Mitteln mit antiproliferativer Wirkung.

Europäisches Patentamt

# EUROPÄISCHER RECHERCHENBERICHT

Nummer der Anmeldung
EP 99 11 2223

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | EP 0 251 194 A (BOEHRINGER INGELHEIM KG ) 7. Januar 1988 (1988-01-07) * Ansprüche * | 1-22 | C07D217/18 C07D471/04 C07D495/04 A61K31/47 A61K31/435 C07D217/14 //(C07D471/04, 221:00, 209:00), (C07D495/04, 333:00,221:00) |
| D,X | EP 0 037 934 A (C.H.BOEHRINGER SOHN) 21. Oktober 1981 (1981-10-21) * Ansprüche * | 1-22 | |
| X | EP 0 288 048 A (BOEHRINGER INGELHEIM KG ) 26. Oktober 1988 (1988-10-26) * Ansprüche 1-11 * | 1-22 | |
| A | EP 0 358 957 A (BOEHRINGER INGELHEIM KG ) 21. März 1990 (1990-03-21) * Ansprüche * | 1-22 | |
| P,A | WO 92 11010 A (BOEHRINGER INGELHEIM KG ) 9. Juli 1992 (1992-07-09) * Ansprüche * | 1-22 | |

RECHERCHIERTE SACHGEBIETE (Int.Cl.6)
C07D
A61K

Der vorliegende Recherchenbericht wurde für alle Patentansprüche erstellt

| Recherchenort | Abschlußdatum der Recherche | Prüfer |
|---|---|---|
| DEN HAAG | 27. Juli 1999 | Henry, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 99 11 2223

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-07-1999

| Im Recherchenbericht angeführtes Patentdokument | Datum der Veröffentlichung | Mitglied(er) der Patentfamilie | Datum der Veröffentlichung |
|---|---|---|---|
| EP 0251194 A | 07-01-1988 | DE 3621413 A | 07-01-1988 |
| | | AT 87619 T | 15-04-1993 |
| | | AU 598558 B | 28-06-1990 |
| | | AU 7477587 A | 07-01-1988 |
| | | DD 264429 A | 01-02-1989 |
| | | DE 3785093 A | 06-05-1993 |
| | | DK 323887 A | 27-12-1987 |
| | | ES 2053471 T | 01-08-1994 |
| | | FI 872827 A | 27-12-1987 |
| | | FI 923048 A | 01-07-1992 |
| | | FI 933146 A | 09-07-1993 |
| | | GR 3007545 T | 31-08-1993 |
| | | IE 59546 B | 09-03-1994 |
| | | JP 63258458 A | 25-10-1988 |
| | | MX 9203623 A | 01-09-1992 |
| | | PT 85169 A,B | 01-07-1987 |
| | | YU 119887 A | 31-10-1988 |
| EP 0037934 A | 21-10-1981 | DE 3013906 A | 15-10-1981 |
| | | AU 6938481 A | 29-10-1981 |
| | | DK 164281 A | 12-10-1981 |
| | | FI 811123 A | 12-10-1981 |
| | | GB 2074159 A | 28-10-1981 |
| | | GR 74904 A | 12-07-1984 |
| | | JP 56158765 A | 07-12-1981 |
| | | NZ 196788 A | 10-05-1983 |
| | | PT 72832 A,B | 01-05-1981 |
| | | US 4322418 A | 30-03-1982 |
| | | YU 94181 A | 30-04-1983 |
| | | ZA 8102391 A | 29-12-1982 |
| EP 0288048 A | 26-10-1988 | DE 3713743 A | 17-11-1988 |
| | | DE 3718570 A | 15-12-1988 |
| | | AT 107921 T | 15-07-1994 |
| | | AU 609121 B | 26-04-1991 |
| | | AU 1509088 A | 27-10-1988 |
| | | CA 1330798 A | 19-07-1994 |
| | | DE 3850437 D | 04-08-1994 |
| | | DK 219488 A | 25-10-1988 |
| | | ES 2055717 T | 01-09-1994 |
| | | FI 881889 A,B, | 25-10-1988 |
| | | FI 935966 A | 31-12-1993 |
| | | HU 208673 B | 28-12-1993 |
| | | JP 2669468 B | 27-10-1997 |
| | | JP 63280069 A | 17-11-1988 |
| | | MX 11218 A | 01-11-1993 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 99 11 2223

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-07-1999

| Im Recherchenbericht angeführtes Patentdokument | Datum der Veröffentlichung | Mitglied(er) der Patentfamilie | Datum der Veröffentlichung |
|---|---|---|---|
| EP 0288048 A | | NO 174548 B | 14-02-1994 |
| | | PH 26225 A | 01-04-1992 |
| | | PT 87300 A,B | 01-05-1988 |
| | | YU 80488 A | 30-04-1990 |
| | | PH 25927 A | 19-12-1991 |
| | | SU 1681724 A | 30-09-1991 |
| EP 0358957 A | 21-03-1990 | DE 3827727 A | 22-02-1990 |
| | | AU 637767 B | 10-06-1993 |
| | | AU 3992589 A | 22-02-1990 |
| | | DD 285595 A | 19-12-1990 |
| | | DD 297640 A | 16-01-1992 |
| | | DD 295379 A | 31-10-1991 |
| | | DE 8817109 U | 26-11-1992 |
| | | DK 399789 A | 17-02-1990 |
| | | FI 893832 A | 17-02-1990 |
| | | FI 921541 A | 08-04-1992 |
| | | JP 2160767 A | 20-06-1990 |
| | | MX 9203260 A | 01-07-1992 |
| | | PL 161521 B | 30-07-1993 |
| | | PT 91451 A | 08-03-1990 |
| WO 9211010 A | 09-07-1992 | DE 4041482 A | 25-06-1992 |
| | | AT 177948 T | 15-04-1999 |
| | | AU 666107 B | 01-02-1996 |
| | | AU 9080291 A | 22-07-1992 |
| | | CA 2098917 A | 23-06-1992 |
| | | CZ 9301213 A | 16-02-1994 |
| | | CZ 9502846 A | 16-07-1997 |
| | | DE 9017900 U | 28-01-1993 |
| | | DE 59109115 D | 29-04-1999 |
| | | EP 0563128 A | 06-10-1993 |
| | | EP 0781556 A | 02-07-1997 |
| | | ES 2129040 T | 01-06-1999 |
| | | FI 932709 A | 14-06-1993 |
| | | HR 940721 A | 31-12-1996 |
| | | HU 74639 A | 28-01-1997 |
| | | IL 100450 A | 10-01-1997 |
| | | JP 6504277 T | 19-05-1994 |
| | | MX 9102765 A | 31-03-1994 |
| | | NO 179515 B | 15-07-1996 |
| | | NZ 241140 A | 24-06-1997 |
| | | PL 172262 B | 29-08-1997 |
| | | PT 99901 A | 31-12-1992 |
| | | SG 43064 A | 17-10-1997 |
| | | SI 9111975 A | 30-06-1997 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 99 11 2223

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-07-1999

| Im Recherchenbericht angeführtes Patentdokument | Datum der Veröffentlichung | Mitglied(er) der Patentfamilie | Datum der Veröffentlichung |
|---|---|---|---|
| WO 9211010 A | | SK 61793 A | 12-01-1994 |
| | | US 5665729 A | 09-09-1997 |
| | | US 5686462 A | 11-11-1997 |

EPO FORM P0461

Für nähere Einzelheiten zu diesem Anhang : siehe Amtsblatt des Europäischen Patentamts, Nr.12/82